(19) **Europäisches Patentamt / European Patent Office / Office européen des brevets**

(11) **EP 4 660 306 A1**

(12) # EUROPEAN PATENT APPLICATION
published in accordance with Art. 153(4) EPC

(43) Date of publication:
**10.12.2025 Bulletin 2025/50**

(21) Application number: **24750309.7**

(22) Date of filing: **30.01.2024**

(51) International Patent Classification (IPC):
***C12N 15/11*** *(2006.01)*

(52) Cooperative Patent Classification (CPC):
**C12N 15/11**

(86) International application number:
**PCT/JP2024/002945**

(87) International publication number:
**WO 2024/162360 (08.08.2024 Gazette 2024/32)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **31.01.2023 JP 2023013290**

(71) Applicant: **Synplogen Co., Ltd.
Kobe-shi, Hyogo 650-0047 (JP)**

(72) Inventors:
• **HOSODA, Nao
Kobe-shi, Hyogo 650-0047 (JP)**

• **HAYASHI, Kentaro
Kobe-shi, Hyogo 650-0047 (JP)**
• **TSUGE, Kenji
Kobe-shi, Hyogo 650-0047 (JP)**

(74) Representative: **Vossius & Partner
Patentanwälte Rechtsanwälte mbB
Siebertstraße 3
81675 München (DE)**

Remarks:
The complete document including Reference
Table(s) and the Sequence Listing(s) can be
downloaded from the EPO website

(54) **ARTIFICIALLY SYNTHESIZED NUCLEIC ACID FOR INCREASING PROTEIN EXPRESSION**

(57) The present disclosure provides a nucleic acid construct capable of regulating protein expression. Specifically, the present disclosure provides a nucleic acid construct including a 5' untranslated region (UTR) and a 3' UTR that are at least partially complementary to each other, wherein at least one of UTRs includes a non-complementary region and a complementary region with respect to the other UTR, and when all of the respective lengths of the non-complementary regions are of 1 base, a degree of complementarity of the 3' UTR to the 5' UTR is greater than 75%.

EP 4 660 306 A1

## Description

Technical Field

The present disclosure provides an artificially synthesized nucleic acid for increasing protein expression.

Background Art

mRNA medicines are important modalities for use in COVID-19 vaccines, cancer vaccines, and disease therapeutics. The medicine exerts its therapeutic effect by delivering mRNA into cells, thereby inducing the expression of a protein which is an active ingredient. It does not need to be transported into the nucleus and a risk of genome integration is low, and therefore, the medicine is considered to have a high level of safety.

Techniques such as codon optimization and sequence optimization for enhancing translation efficiency have been developed. A plasmid encoding a target mRNA is used as a raw material, and after linearization, transcription, addition of a cap, and removal of template DNA with DNase are performed in a test tube. There is also a method of enzymatically adding a polyA tail later. In addition, a translation promotion mechanism by circularizing mRNA is known.

Summary of Invention

Solution to Problem

The present inventors have newly found that in a nucleic acid construct, protein expression can be regulated by a degree of complementarity between a 3' untranslated region and a 5' untranslated region and specific patterns of a complementary region and a non-complementary region. Thus, the present disclosure provides a nucleic acid construct including a 5' untranslated region (UTR) and a 3' UTR that are at least partially complementary to each other, in which the nucleic acid construct has a specific complementary pattern.

Accordingly, the present disclosure provides the following.

[Item X1]

A nucleic acid construct including a 5' untranslated region (UTR) and a 3' UTR that are at least partially complementary to each other, wherein at least one of UTRs includes a non-complementary region and a complementary region with respect to the other UTR, and when all of the respective lengths of the non-complementary regions are one base substitutions, a degree of complementarity of the 3' UTR to the 5' UTR is greater than 75%.

[Item X2]

The nucleic acid construct according to the above item, wherein the base of the non-complementary region is subject to substitution or removal.

[Item X2A]

The nucleic acid construct according to any one of the above items, wherein the non-complementary regions are all of substitutions.

[Item X2B]

The nucleic acid construct according to any one of the above items, wherein the non-complementary regions are all of substitutions and each has a length of one base.

[Item X2C]

The nucleic acid construct according to any one of the above items, wherein the non-complementary regions are all of removals.

[Item X3]

The nucleic acid construct according to any one of the above items, wherein at least one of the non-complementary regions includes a length of 2 or more bases.

[Item X4]

The nucleic acid construct according to any one of the above items, wherein the length of at least one of the non-complementary regions is of 2 bases or 3 bases.

[Item X5]

The nucleic acid construct according to any one of the above items, wherein the length of at least one of the complementary regions is of 5 or more bases.

[Item X6]

The nucleic acid construct according to any one of the above items, wherein the length of at least one of the complementary regions is of 5 to 11 bases.

[Item X7]

The nucleic acid construct according to any one of the above items, wherein the length of the non-complementary region is of 2 bases, and the lengths of the complementary regions are each independently of 5 to 7 bases.

[Item X8]

The nucleic acid construct according to any one of the above items, wherein the length of the non-complementary region is of 3 bases, and the lengths of the complementary regions are each independently of 8 to 11 bases.

[Item X9]

The nucleic acid construct according to any one of the above items, wherein all of the respective lengths of the non-complementary regions are each of 1 or more bases, and the degree of complementarity is greater than 75%.

[Item X10]

The nucleic acid construct according to any one of the above items, wherein the degree of complementarity is 80% to 90%.

[Item X11]

The nucleic acid construct according to any one of the above items, wherein the degree of complementarity is 81% to 89%.

[Item X12]

The nucleic acid construct according to any one of the above items, wherein at least one of the non-complementary regions is 1 base.

[Item X13]

The nucleic acid construct according to any one of the above items, wherein the lengths of the complementary regions are each independently of 3 to 11 bases.

[Item X14]

The nucleic acid construct according to any one of the above items, wherein the non-complementary region is 1 base, and the lengths of the complementary regions are each independently of 3 to 7 bases.

[Item X15]

The nucleic acid construct according to any one of the above items, wherein the length of the non-complementary region is of 2 bases, and the lengths of the complementary regions are each independently of 5 to 7 bases.

[Item X16]

The nucleic acid construct according to any one of the above items, wherein the length of the non-complementary region is of 3 bases, and the lengths of the complementary regions are each independently of 8 to 11 bases.

[Item X17]

A nucleic acid construct including a 5' untranslated region (UTR) and a 3' UTR that are at least partially complementary to each other, wherein at least one of UTRs includes a non-complementary region and a complementary region with respect to the other UTR, and the non-complementary region and the complementary region are alternately present.

[Item X17A]

The nucleic acid construct according to any one of the above items, wherein a region other than the alternately present regions (also referred to as a residual region (4)) is 1 or more bases and less than or equal to a base length of the complementary region.

[Item X17B]

The nucleic acid construct according to any one of the above items, wherein when the non-complementary region (2) is 1 base, the complementary region (3) is 3 to 6 bases in length in a case of base substitution and 3 to 9 bases in length in a case of base removal.

[Item X18]

The nucleic acid construct according to any one of the above items, wherein when the non-complementary region is 2 bases, the complementary region (3) is 5 to 7 bases in length in a case of base substitution and 5 to 11 bases in length in a case of base removal.

[Item X19]

The nucleic acid construct according to any one of the above items, wherein when the non-complementary region is 3 bases, the complementary region is 8 to 9 bases in length in a case of base substitution and 8 to 11 bases in length in a case of base removal.

[Item X19A]

The nucleic acid construct according to any one of the above items, wherein the non-complementary region (2) is 2 bases or 3 bases.

[Item X19B]

The nucleic acid construct according to any one of the above items, wherein all of the respective lengths of the non-complementary regions are of 1 or more bases, and the degree of complementarity is greater than 75%.

[Item X19C]

The nucleic acid construct according to any one of the above items, wherein the degree of complementarity is 80% to 90%.

[Item X19D]

The nucleic acid construct according to any one of the above items, wherein the degree of complementarity is 81% to 89%.

[Item X20]

The nucleic acid construct according to any one of the above items, wherein either the 5' UTR or the 3' UTR does not include a non-complementary region.

[Item X21]

The nucleic acid construct according to any one of the above items, wherein a change in free energy ($\Delta$G) is a predetermined value or a range thereof.

[Item X22]

The nucleic acid construct according to any one of the above items, wherein the predetermined value or the range thereof is a value or range of a change in free energy in a range of a rate of increase of 0% to 60% with respect to an average value of a maximum value and a minimum value of the change in free energy ($\Delta$G).

[Item X23]

The nucleic acid construct according to any one of the above items, wherein the predetermined value or the range thereof is a value or range of a change in free energy in a range of a rate of increase of 10% to 50% with respect to an average value of a maximum value and a minimum value of the change in free energy ($\Delta$G).

[Item X24]

A method of producing a nucleic acid construct, the nucleic acid construct including a 5' untranslated region (UTR) and a 3' UTR that are at least partially complementary to each other, in which at least one of UTRs includes a non-complementary region and a complementary region to the other UTR, the method including: a step of designing a plurality of candidate nucleic acid constructs; a step of calculating a change in free energy ($\Delta$G) for the plurality of designed candidate nucleic acid constructs; a step of selecting a candidate nucleic acid construct of which a change in free energy ($\Delta$G) is a predetermined value or a range thereof; and a step of optionally measuring an expression level of the selected candidate nucleic acid construct in cells.

[Item X25]

A method of producing a nucleic acid construct, the nucleic acid construct including a 5' untranslated region (UTR) and a 3' UTR that are at least partially complementary to each other, in which at least one of UTRs includes a non-complementary region and a complementary region to the other UTR, the method including: a step of designing a plurality of candidate nucleic acid constructs; a step of calculating a change in free energy ($\Delta$G) for the plurality of designed candidate nucleic acid constructs; a step of selecting a candidate nucleic acid construct having a value of a change in free energy in a range of a rate of increase of 0% to 60% with respect to an average value of a maximum value and a minimum value of the change in free energy ($\Delta$G); and a step of optionally measuring an expression level of the selected candidate nucleic acid construct in cells.

[Item X26]

The method according to any one of the above items, wherein the rate of increase with respect to the average value is 10% to 50%.

[Item X27]

The nucleic acid construct according to any one of the above items, wherein the nucleic acid construct is produced by the method according to any one of any one of the above items.
In addition, the present disclosure also provides the following.

(Item 1)

A nucleic acid construct including a 5' untranslated region (UTR) and a 3' UTR that are at least partially complementary to each other, wherein at least one of UTRs includes a non-complementary region and a complementary region with respect to the other UTR, and when all of the respective lengths of the non-complementary regions are of 1 base, a degree of complementarity of the 3' UTR to the 5' UTR is greater than 75%.

(Item 2)

The nucleic acid construct according to Item 1, wherein at least one of the non-complementary regions comprises a length of 2 or more bases.

(Item 3)

The nucleic acid construct according to Item 1 or 2, wherein the respective length(s) of at least one of the non-complementary regions is/are of 2 bases or 3 bases.

(Item 4)

The nucleic acid construct according to any one of Items 1 to 3, wherein the length of at least one of the complementary regions is of 5 or more bases.

(Item 5)

The nucleic acid construct according to any one of Items 1 to 3, wherein the length of at least one of the complementary regions is of 5 to 11 bases.

(Item 6)

The nucleic acid construct according to Item 1, wherein the length of the non-complementary region is of 2 bases, and the lengths of the complementary region(s) is/are each independently of 5 to 7 bases.

(Item 7)

The nucleic acid construct according to Item 1, wherein the length of the non-complementary region is of 3 bases, and the lengths of the complementary region(s) is/are each independently of 8 to 11 bases.

(Item 8)

The nucleic acid construct according to Item 1, wherein all of the respective lengths of the non-complementary regions are of 1 or more bases, and the degree of complementarity is greater than 75%.

(Item 9)

The nucleic acid construct according to Item 8, wherein the degree of complementarity is 80% to 90%.

(Item 10)

The nucleic acid construct according to Item 8, wherein the degree of complementarity is 81% to 89%.

(Item 11)

The nucleic acid construct according to Item 7, wherein at least one of the non-complementary regions is 1 base.

(Item 12)

The nucleic acid construct according to any one of Items 8 to 11, wherein the respective lengths of the complementary regions are each independently of 3 to 11 bases.

(Item 13)

The nucleic acid construct according to Item 8, wherein the non-complementary region is 1 base, and the lengths of the complementary regions are each independently of 3 to 7 bases.

(Item 14)

The nucleic acid construct according to Item 8, wherein the length of the non-complementary region is of 2 bases, and the lengths of the complementary regions are each independently of 5 to 7 bases.

(Item 15)

The nucleic acid construct according to Item 8, wherein the length of the non-complementary region is of 3 bases, and the lengths of the complementary regions are each independently of 8 to 11 bases.

(Item 16)

The nucleic acid construct according to Item 1, wherein either the 5' UTR or the 3' UTR does not include a non-complementary region.

(Item 17)

The nucleic acid construct according to any one of Items 1 to 16, wherein a change in free energy ($\Delta$G) is a predetermined value or a range thereof.

(Item 18)

The nucleic acid construct according to Item 17, wherein the predetermined value or the range thereof is a value or range of a change in free energy in a range of a rate of increase of 0% to 60% with respect to an average value of a maximum value and a minimum value of the change in free energy ($\Delta$G).

(Item 19)

The nucleic acid construct according to Item 17, wherein the predetermined value or the range thereof is a value or range of a change in free energy in a range of a rate of increase of 10% to 50% with respect to an average value of a maximum value and a minimum value of the change in free energy ($\Delta$G).

(Item 20)

A method of producing a nucleic acid construct, the nucleic acid construct including a 5' untranslated region (UTR) and a 3' UTR that are at least partially complementary to each other, in which at least one of UTRs includes a non-complementary region and a complementary region to the other UTR, the method including: a step of designing a plurality of candidate nucleic acid constructs; a step of calculating a change in free energy ($\Delta$G) for the plurality of designed candidate nucleic acid constructs; a step of selecting a candidate nucleic acid construct of which a change in free energy ($\Delta$G) is a predetermined value or a range thereof; and a step of optionally measuring an expression level of the selected candidate nucleic acid construct in cells.

(Item 21)

A method of producing a nucleic acid construct, the nucleic acid construct including a 5' untranslated region (UTR) and a 3' UTR that are at least partially complementary to each other, in which at least one of UTRs includes a non-complementary region and a complementary region to the other UTR, the method including: a step of designing a plurality of candidate nucleic acid constructs; a step of calculating a change in free energy ($\Delta$G) for the plurality of designed candidate nucleic acid constructs; a step of selecting a candidate nucleic acid construct having a value of a change in free energy in a range of a rate of increase of 0% to 60% with respect to an average value of a maximum value and a minimum value of the change in free energy ($\Delta$G); and a step of optionally measuring an expression level of the selected candidate nucleic acid construct in cells.

(Item 22)

The method according to Item 21, wherein the rate of increase with respect to the average value is 10% to 50%.

(Item 23)

The nucleic acid construct according to any one of Items 1 to 19, wherein the nucleic acid construct is produced by the method according to any one of Items 20 to 22. Advantageous Effects of Invention

The nucleic acid construct of the present disclosure is an improved nucleic acid construct that exhibits a high expression level of a protein compared to a conventional nucleic acid construct.

Brief Description of Drawings

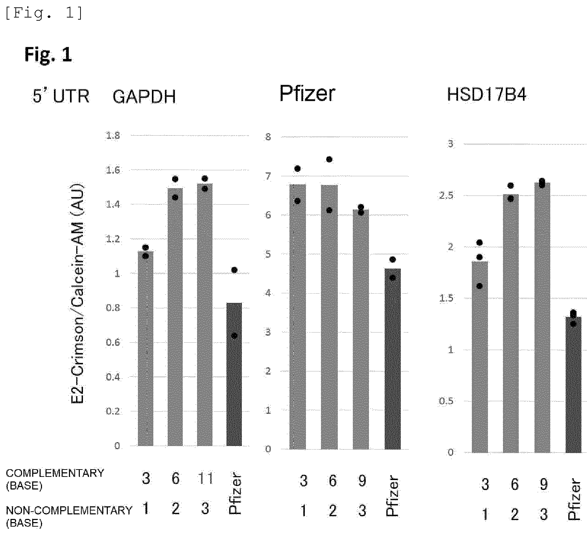

[FIG. 1] FIG. 1 illustrates the results of examining an expression level of mRNA in which a 5' UTR is a GAPDH gene sequence (left side of the drawing), a Pfizer sequence (messenger RNA encoding the full-length SARS-CoV-2 spike glycoprotein Sept. 2020 document 11889; see the material of the 19th Health Sciences Council of the Subcommittee on Immunization and Vaccination (https://www.mhlw.go.jp/stf/shingi2/0000192554_00004.html)) (center of the drawing), or an HSD17B4 gene sequence (right of the drawing). For comparison, mRNAs in which the 5' UTR and 3' UTR were both the Pfizer sequences were used. A ratio of an E2Crimson fluorescence measurement value to a Calcein fluorescence measurement value was defined as a relative expression level of E2Crimson protein in each mRNA. A plurality of samples were measured for each mRNA; the average value is shown in a bar graph, and the individual values of each sample are shown as black dots.

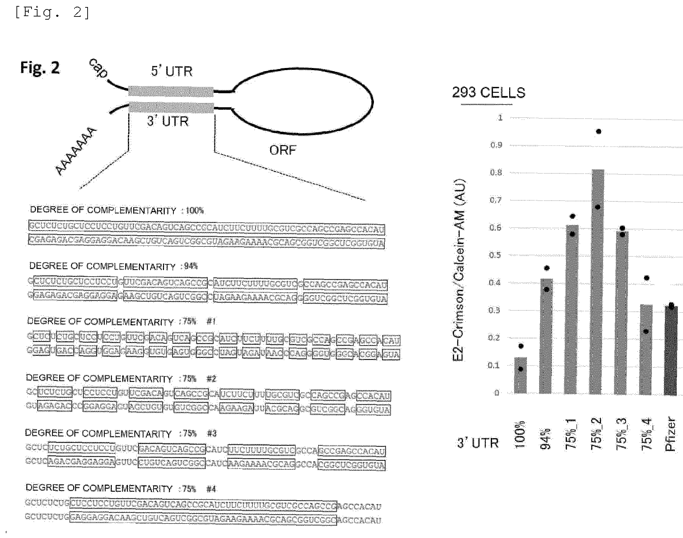

[FIG. 2] FIG. 2 illustrates the results of examining an expression level of mRNA in which a 5' UTR is a GAPDH gene sequence, and a 3' UTR is a sequence having a degree of complementarity of 100%, 94%, or 75% to the 5' UTR. The upper left of the drawing schematically illustrates the mRNA structure. The lower left illustrates base sequences of the 5' UTR and 3' UTR, and in each sequence, the upper row shows the 5' UTR sequence with the 5' end on the left side, and the lower row shows the 3' UTR sequence with the 5' end on the right side. Description of ORF is omitted. Complementary bases between the 5' UTR and the 3' UTR are indicated by enclosures. The right side of the drawing is a graph showing the results of examining an expression level in 293 cells for each mRNA. For comparison, mRNAs in which both the 5' UTR and 3' UTR were the Pfizer sequences were used. A ratio of an E2Crimson fluorescence measurement value to a Calcein fluorescence measurement value was defined as a relative expression level of E2Crimson protein in each mRNA. A plurality of samples were measured for each mRNA; the average value is shown in a bar graph, and the individual values of each sample are shown as black dots.

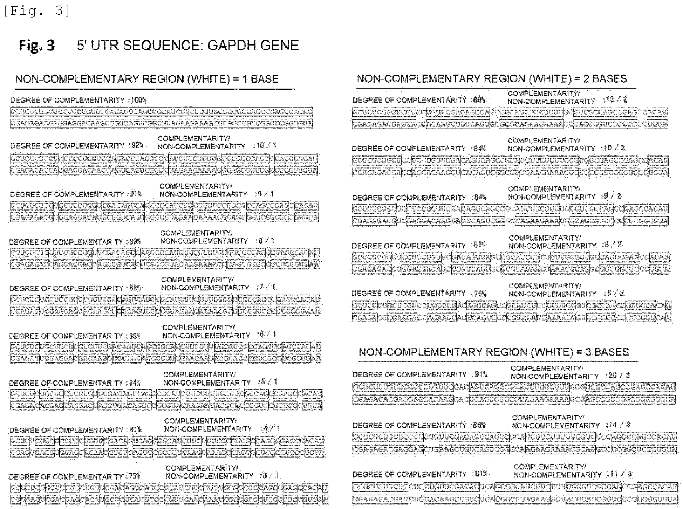

[FIG. 3] FIG. 3 illustrates base sequences of 5' UTR and 3' UTR of various mRNAs in which the 5' UTR is a GAPDH gene sequence and the 3' UTR is partially complementary to the 5' UTR. The non-complementary regions are classified according to the number of bases. In each sequence, the upper row shows the 5' UTR sequence with the 5' end on the left side, and the lower row shows the 3' UTR sequence with the 5' end on the right side. Description of ORF is omitted. Complementary bases between the 5' UTR and the 3' UTR are indicated by enclosures.

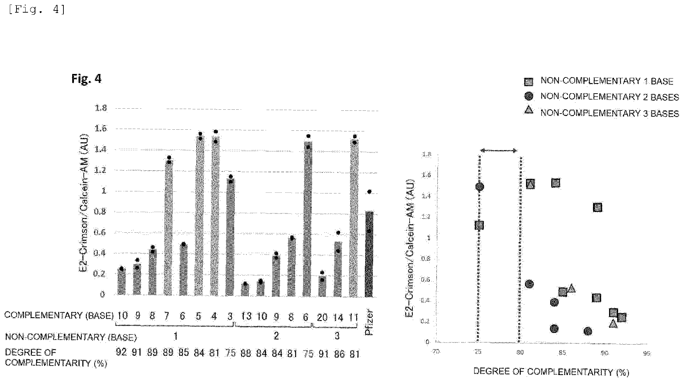

[FIG. 4] FIG. 4 is a graph showing the results of examining an expression level in 293 cells for mRNA having a GAPDH gene sequence as a 5' UTR and various sequences having partial complementarity to the 5' UTR as a 3' UTR (left). The right side of the drawing shows a distribution of the degree of complementarity with respect to the relative expression level (ratio of E2Crimson fluorescence measurement value to Calcein fluorescence measurement value) in the left side of the drawing. For comparison, mRNAs in which both the 5' UTR and 3' UTR were the Pfizer sequences were used. A ratio of an E2Crimson fluorescence measurement value to a Calcein fluorescence measurement value was defined as a relative expression level of E2Crimson protein in each mRNA. A plurality of samples were measured for each mRNA; the average value is shown in a bar graph, and the individual values of each sample are shown as black dots.

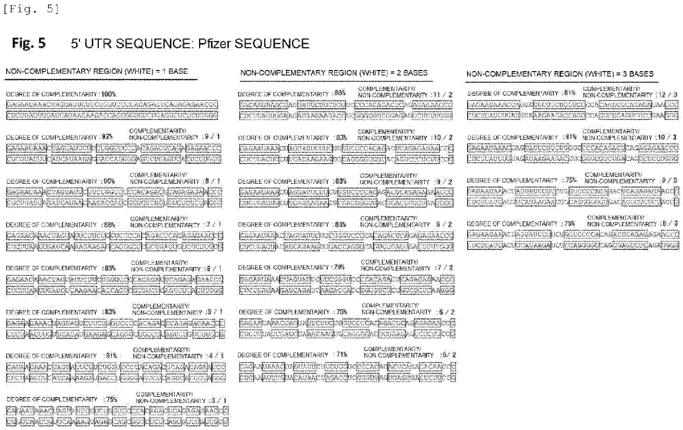

[FIG. 5] FIG. 5 illustrates base sequences of 5' UTR and 3' UTR of various mRNAs in which the 5' UTR is a Pfizer sequence and the 3' UTR is partially complementary to the 5' UTR. The non-complementary regions are classified according to the number of bases. In each sequence, the upper row shows the 5' UTR sequence with the 5' end on the left side, and the lower row shows the 3' UTR sequence with the 5' end on the right side. Description of ORF is omitted. Complementary bases between the 5' UTR and the 3' UTR are indicated by enclosures.

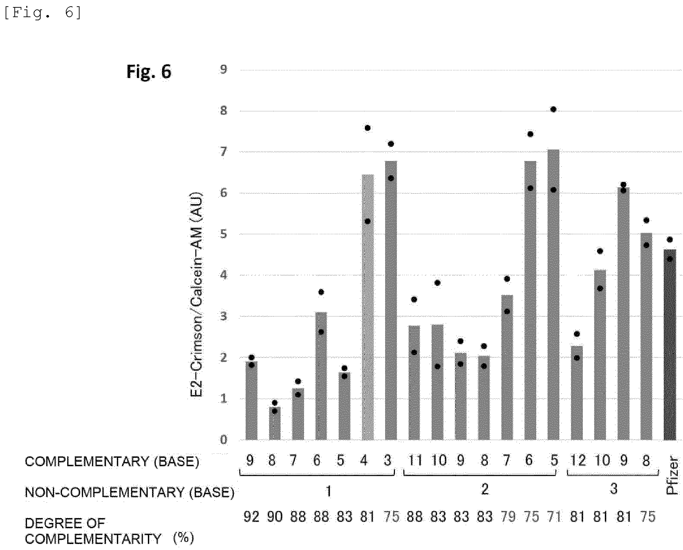

[FIG. 6] FIG. 6 is a graph showing the results of examining an expression level in 293 cells for mRNA having a Pfizer sequence as a 5' UTR and various sequences having partial complementarity to the 5' UTR as a 3' UTR. For comparison, mRNAs in which both the 5' UTR and 3' UTR were the Pfizer sequences were used. A ratio of an E2Crimson fluorescence measurement value to a Calcein fluorescence measurement value was defined as a relative expression level of E2Crimson protein in each mRNA. A plurality of samples were measured for each mRNA; the average value is shown in a bar graph, and the individual values of each sample are shown as black dots.

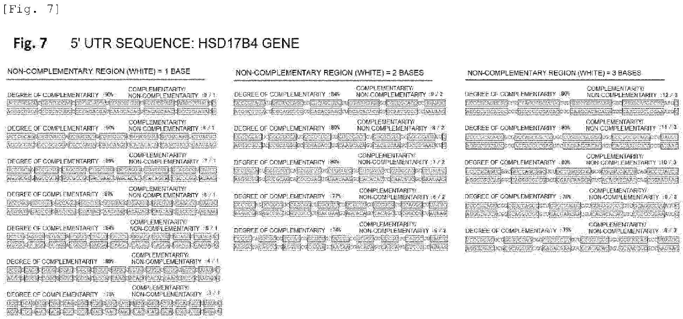

[FIG. 7] FIG. 7 illustrates base sequences of 5' UTR and 3' UTR of various mRNAs in which the 5' UTR is an HSD17B4 gene sequence and the 3' UTR is partially complementary to the 5' UTR. The non-complementary regions are classified according to the number of bases. In each sequence, the upper row shows the 5' UTR sequence with the 5' end on the left side, and the lower row shows the 3' UTR sequence with the 5' end on the right side. Description of ORF is omitted. Complementary bases between the 5' UTR and the 3' UTR are indicated by enclosures.

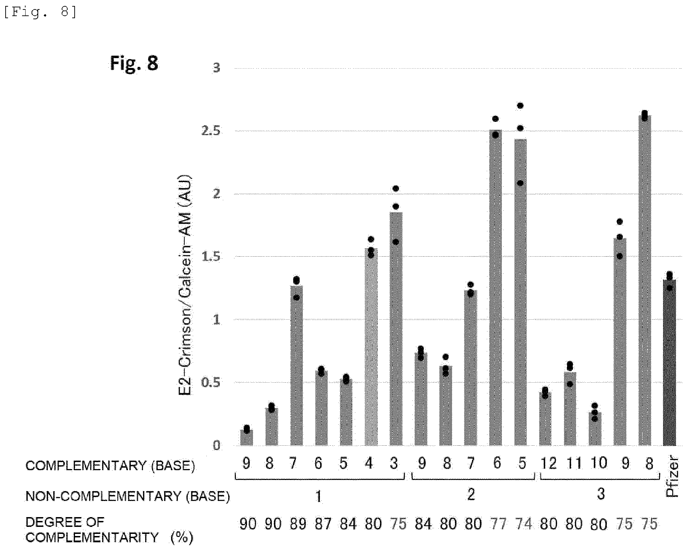

[FIG. 8] FIG. 8 is a graph showing the results of examining an expression level in 293 cells for mRNA having an HSD17B4 gene sequence as a 5' UTR and various sequences having partial complementarity to the 5' UTR as a 3' UTR. For comparison, mRNAs in which both the 5' UTR and 3' UTR were the Pfizer sequences were used. A ratio of an E2Crimson fluorescence measurement value to a Calcein fluorescence measurement value was defined as a relative expression level of E2Crimson protein in each mRNA. A plurality of samples were measured for each mRNA; the average value is shown in a bar graph, and the individual values of each sample are shown as black dots.

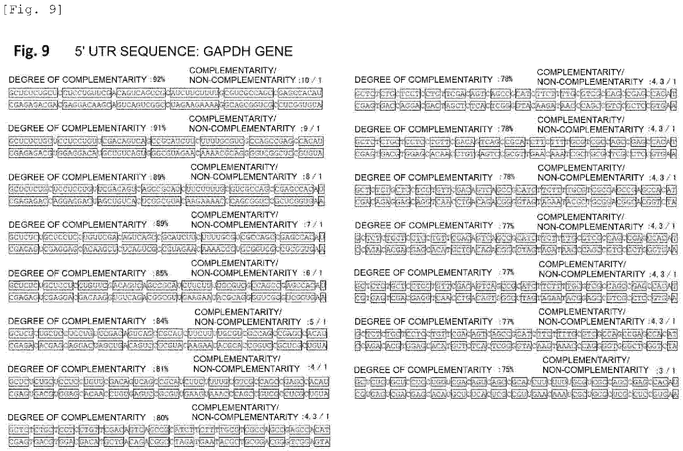

[FIG. 9] FIG. 9 illustrates base sequences of 5' UTR and 3' UTR of various mRNAs in which the 5' UTR is a GAPDH gene sequence and the 3' UTR is partially complementary to the 5' UTR. The sequences are listed in descending order of the degree of complementarity. In each sequence, the upper row shows the 5' UTR sequence with the 5' end on the left side, and the lower row shows the 3' UTR sequence with the 5' end on the right side. Description of ORF is omitted. Complementary bases between the 5' UTR and the 3' UTR are indicated by enclosures.

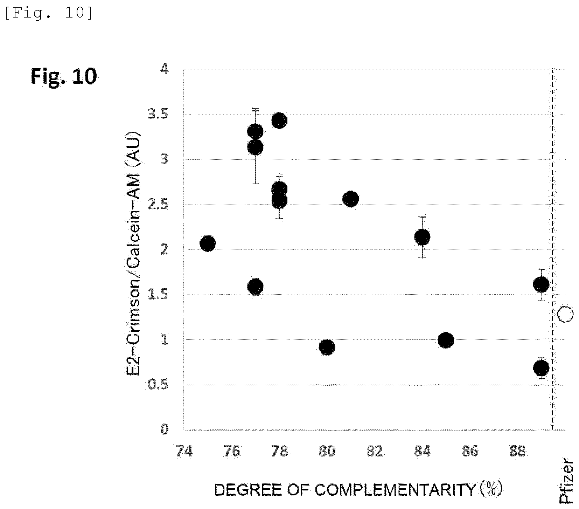

[FIG. 10] FIG. 10 illustrates the results of examining an expression level in 293 cells for mRNA having a GAPDH gene sequence as a 5' UTR and various sequences having partial complementarity to the 5' UTR as a 3' UTR. For comparison, mRNAs in which both the 5' UTR and 3' UTR were the Pfizer sequences were used. A ratio of an E2Crimson fluorescence measurement value to a Calcein fluorescence measurement value was defined as a relative expression level of E2Crimson protein in each mRNA. The vertical axis represents the relative expression level, the horizontal axis represents the degree of complementarity of each mRNA, and the results are shown as a scatter plot. Three samples were measured for each mRNA, and the average value is shown as a plot. Furthermore, the standard deviation of these measured values is calculated and shown as the error bar on the plot. In terms of the 5' UTR, mRNA with the GAPDH gene sequence is shown as the black plot and mRNA with the Pfizer sequence is shown as the white plot. The degree of complementarity of mRNA with the maximum expression level was 75% to 80%.

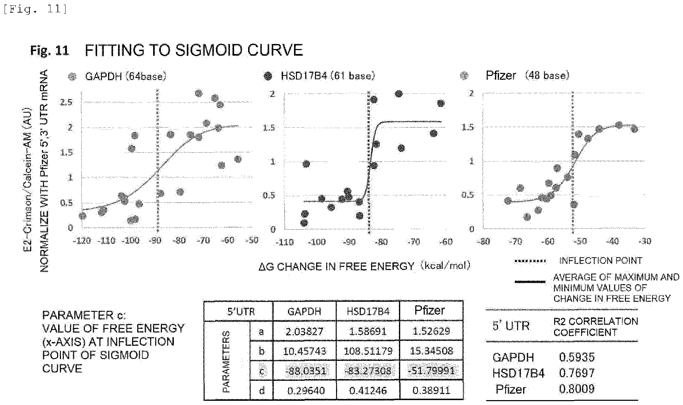

[FIG. 11] FIG. 11 illustrates a correlation between a change in free energy in interaction between a 5' UTR and a 3' UTR and an expression level of each mRNA for mRNA in which a 3' UTR has partial complementarity to a 5' UTR. The expression level was calculated with mRNA of a Pfizer sequence as 1 for both the 5' UTR and the 3' UTR. The change in free energy was obtained by the secondary structure prediction program Mfold (Michael Zuker, Mfold web server for nucleic acid folding and hybridization prediction, Nucleic Acids Res. 2003 Jul 1;31(13):3406-15). The left side, center, and right side of the drawing show the results when the 5' UTRs are those of a GAPDH gene, an HSD17B4 gene, and a Pfizer sequence, respectively. For each 5' UTR, the obtained measured value was fitted to a 4-parameter logistic curve $y = d + (a-d)/(1+ (x/c)^b)$, which is one of the sigmoid curves, to obtain parameters a, b, c, and d and correlation coefficients. The value of the parameter c is the value of the change in free energy that becomes the inflection point on the sigmoid curve. In the graph, a plurality of samples are measured for each mRNA, the average value thereof is plotted, the obtained sigmoid curve is indicated by a solid line, and the value of the change in free energy at an inflection point is indicated by a dashed line.

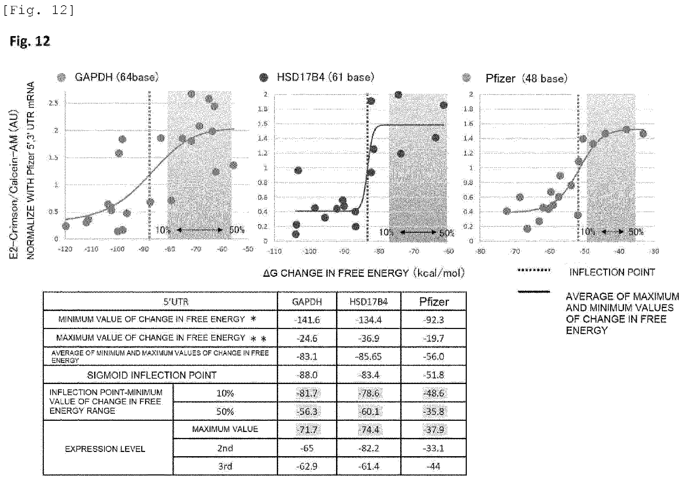

[FIG. 12] FIG. 12 illustrates a possible value of the change in free energy in the interaction between the 5' UTR and the 3' UTR of mRNA having the maximum expression level. The left side, center, and right side of the drawing show the results when the 5' UTRs are those of a GAPDH gene, an HSD17B4 gene, and a Pfizer sequence, respectively. The minimum value of the change in free energy was a value obtained by determining the change in free energy by Mfold for mRNA in which the degree of complementarity between the 5' UTR and the 3' UTR was 100%. The maximum value of the change in free energy was defined as a value of the change in free energy calculated by Mfold for an mRNA in which base substitutions were introduced in the entire 3' UTR to create mismatches with the 5' UTR, where the degree of complementarity between the 5' UTR and 3' UTR was 100%. The relative value of the change in free energy was calculated with the rate of increase (right shift) from the inflection point as an index and, where the difference between the value at the inflection point and the maximum value as 100%. The expression level of mRNA was the highest when the value of the change in free energy represented a rate of increase of 10% or more and 50% or less from the inflection point.

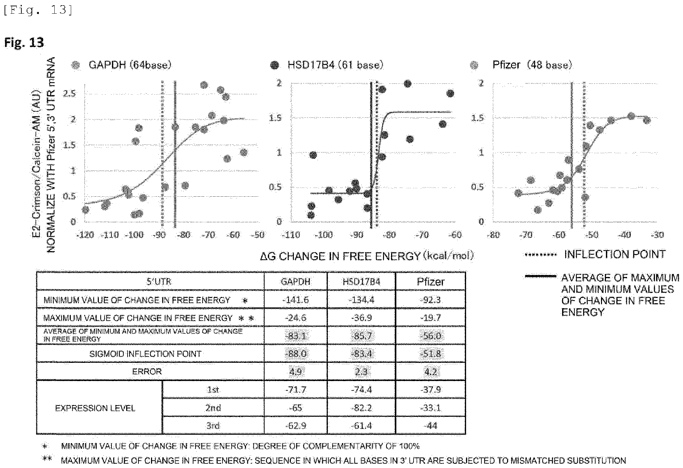

[FIG. 13] FIG. 13 illustrates a method for predicting an expression level of mRNA from 5' UTR and 3' UTR sequences. The maximum value and the minimum value of the change in free energy were obtained in the same manner as in FIG. 12. It was found that the average value of the maximum value and the minimum value of the free energy was consistent with the value of the change in free energy that becomes the inflection point of the sigmoid curve. By using the average value, the inflection point could be predicted with an accuracy of an error of 4.9 kcal/mol or less or 13.1% or less. Similarly to FIG. 12, the error percentage is calculated by taking the difference between the value to be the inflection point and the maximum value of the change in free energy as 100%. Based on the prediction of the inflection point, the change in free energy of the interaction between the 5' UTR and the 3' UTR in mRNA at which the expression level is maximized can be predicted.

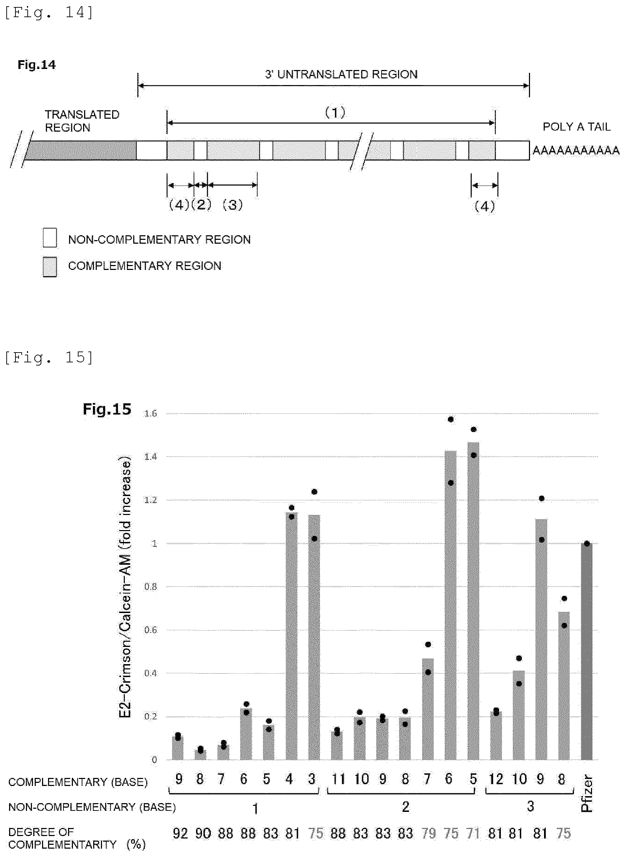

[FIG. 14] FIG. 14 illustrates a schematic diagram of a nucleic acid construct of the present disclosure.

[FIG. 15] FIG. 15 is a graph showing the results of examining the expression level of the mRNA used in FIG. 6 in myoblast cell line C2C12. A ratio of an E2Crimson fluorescence measurement value to a Calcein fluorescence measurement value was defined as a relative expression level of E2Crimson protein in each mRNA. A plurality of samples were measured for each mRNA; the average value is shown in a bar graph, and the individual values of each sample are shown as black dots.

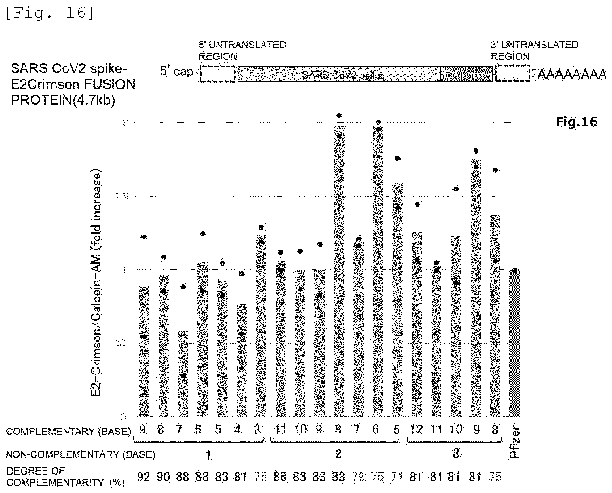

[FIG. 16] FIG. 16 is a graph showing the results of examining an expression level in 293 cells for mRNA having a Pfizer sequence as a 5' UTR, various sequences having partial complementarity with the 5' UTR as a 3' UTR, and SARS CoV2 Spike-E2Crimson fusion protein as ORF. A ratio of an E2Crimson fluorescence measurement value to a Calcein fluorescence measurement value was defined as a relative expression level of E2Crimson protein in each mRNA. A plurality of samples were measured for each mRNA; the average value is shown in a bar graph, and the individual values of each sample are shown as black dots.

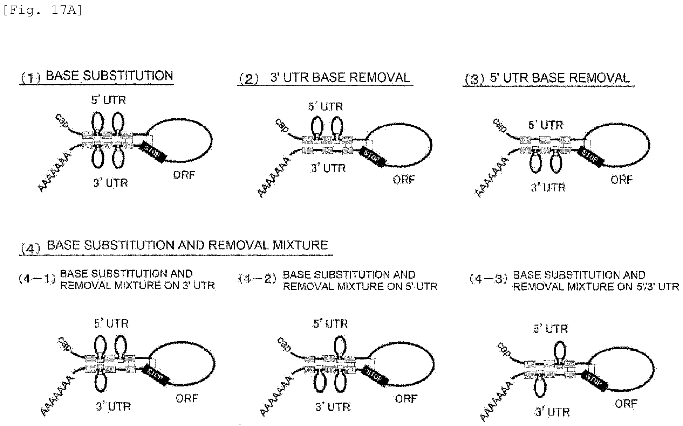

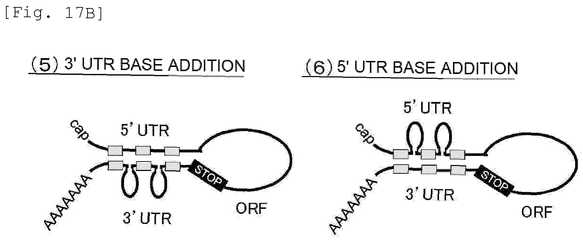

[FIG. 17A] FIG. 17 is a schematic diagram of a concept of substitution and removal related to a method of designing a non-complementary region. FIG. 17 is shown in FIGS. 17A and 17B. FIG. 17(1) illustrates a schematic diagram of mRNA converted into a non-complementary sequence by substituting the base in the complementary sequence. FIG. 17(2) illustrates a schematic diagram of mRNA converted into a non-complementary sequence by removing the base in the complementary sequence. FIG. 17(3) illustrates a schematic diagram of mRNA converted into a non-complementary sequence by removing a base on a 5' UTR in a complementary sequence. FIG. 17(4) illustrates a schematic diagram of mRNA in which there is a mixture of a case in which a complementary sequence is converted into a non-complementary sequence by substituting the base in the complementary sequence and a case in which a complementary sequence is converted into a non-complementary sequence by removing the base. FIG. 17 illustrates a schematic diagram of mRNA in which the base removal and the base substitution are mixed only on the 3' UTR in FIG. 17(4-1), only on the 5' UTR in FIG. 17(4-2), and on either the 5' UTR or the 3' UTR in FIG. 17(4-3).

[FIG. 17B] FIG. 17B is a continuation of FIG. 17A. FIG. 17(5) illustrates a schematic diagram of mRNA converted into a non-complementary sequence by adding a base on a 3' UTR. FIG. 17(6) illustrates a schematic diagram of mRNA converted into a non-complementary sequence by adding a base on a 5' UTR.

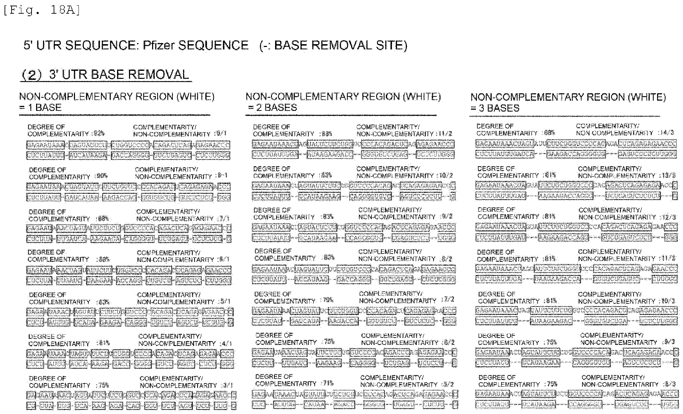

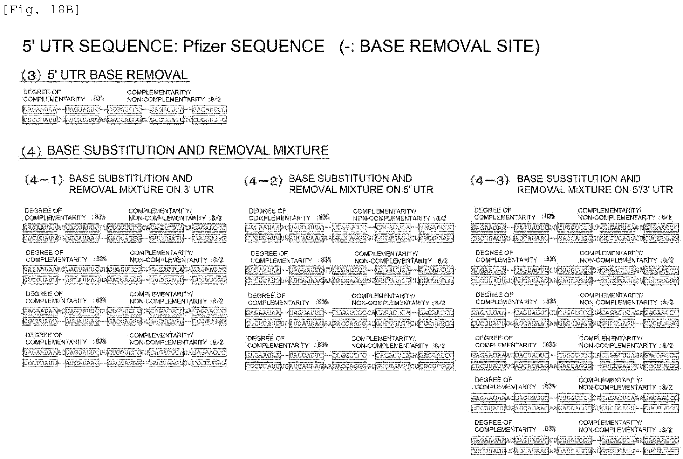

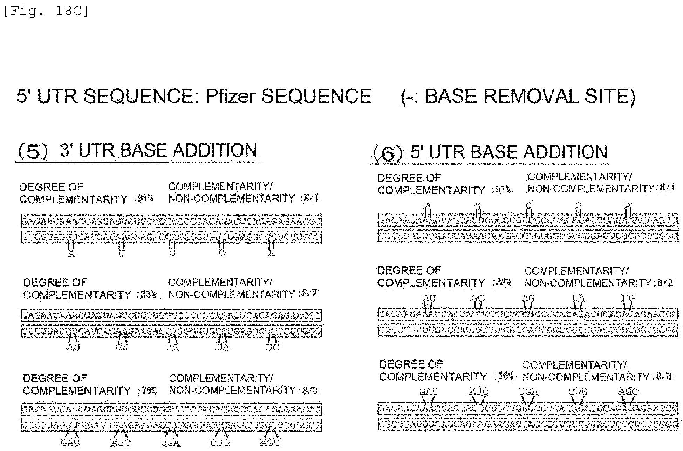

[FIG. 18A] FIG. 18 is divided into FIGS. 18A to 18C, and specifically illustrates base sequences of 5' UTR and 3' UTR of various mRNAs in which the 5' UTR is a Pfizer sequence and the 3' UTR is partially complementary to the 5' UTR for (2) to (6) of the schematic diagrams shown in FIG. 17. FIG. 18(2) illustrates mRNAs converted into a non-complementary sequence by removing the base in the 3' UTR, classified according to the number of bases of the non-complementary region.

[FIG. 18B] FIG. 18B is a continuation of FIG. 18A. FIG. 18(3) illustrates mRNA converted into a non-complementary sequence by removing the base in the 5' UTR. FIG. 18(4) illustrates mRNA in which there is a mixture of a case in which a complementary sequence is converted into a non-complementary sequence by substituting the base in the complementary sequence and a case in which a complementary sequence is converted into a non-complementary sequence by removing the base in the complementary sequence. FIG. 18 illustrates mRNA in which the base removal and the base substitution are mixed only on the 3' UTR in FIG. 18(4-1), only on the 5' UTR in FIG. 18(4-2), and on either the 5' UTR or the 3' UTR in FIG. 18(4-3).

[FIG. 18C] FIG. 18C is a continuation of FIG. 18B. FIG. 18(5) illustrates mRNA in which a non-complementary sequence is created by adding a base in the 3' UTR when the non-complementary region is 1 base, 2 bases, or 3 bases. FIG. 18(6) illustrates mRNA in which a non-complementary sequence is created by adding a base in the 5' UTR when the non-complementary region is 1 base, 2 bases, or 3 bases. In each sequence, the upper row shows the 5' UTR sequence with the 5' end on the left side, and the lower row shows the 3' UTR sequence with the 5' end on the right side. A base indicated by "-" in the lower part indicates a base converted into a non-complementary sequence by removing the base in the complementary sequence. Description of ORF is omitted. Complementary bases between the 5' UTR and the 3' UTR are indicated by enclosures.

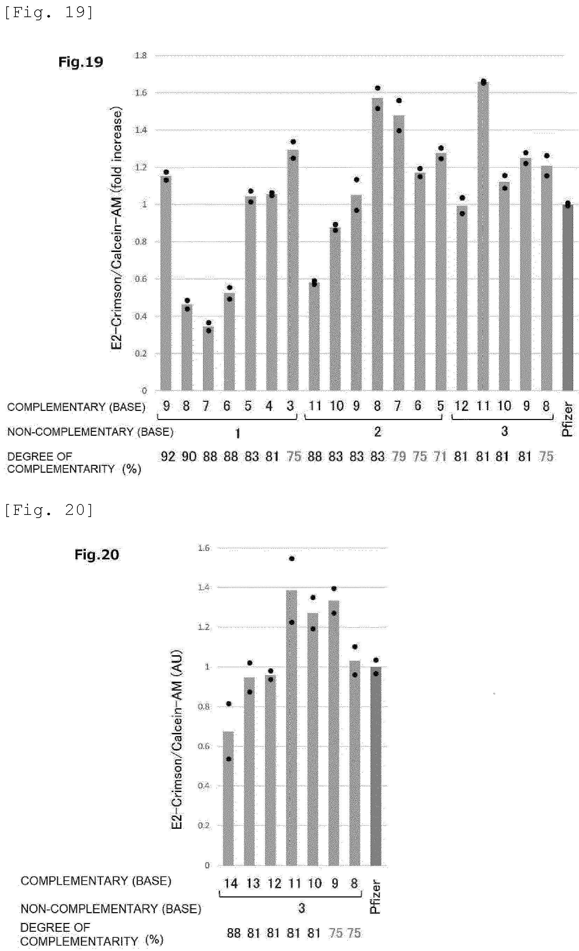

[FIG. 19] FIG. 19 is a graph showing the results of examining an expression level in 293 cells for mRNA having a Pfizer sequence as a 5' UTR and various sequences having partial complementarity to the 5' UTR as a 3' UTR. In these 3' UTRs, the base is removed to form a non-complementary sequence. For comparison, mRNAs in which both the 5' UTR and 3' UTR were the Pfizer sequences were used. A ratio of an E2Crimson fluorescence measurement value to a Calcein fluorescence measurement value was defined as a relative expression level of E2Crimson protein in each mRNA. A plurality of samples were measured for each mRNA; the average value is shown in a bar graph, and the individual values of each sample are shown as black dots.

[FIG. 20] FIG. 20 is a graph showing the results of examining an expression level in 293 cells for mRNA having a Pfizer sequence as a 5' UTR and various sequences having partial complementarity to the 5' UTR as a 3' UTR and including a non-complementary region of 3 bases. A ratio of an E2Crimson fluorescence measurement value to a Calcein fluorescence measurement value was defined as a relative expression level of E2Crimson protein in each mRNA. A plurality of samples were measured for each mRNA; the average value is shown in a bar graph, and the individual values of each sample are shown as black dots.

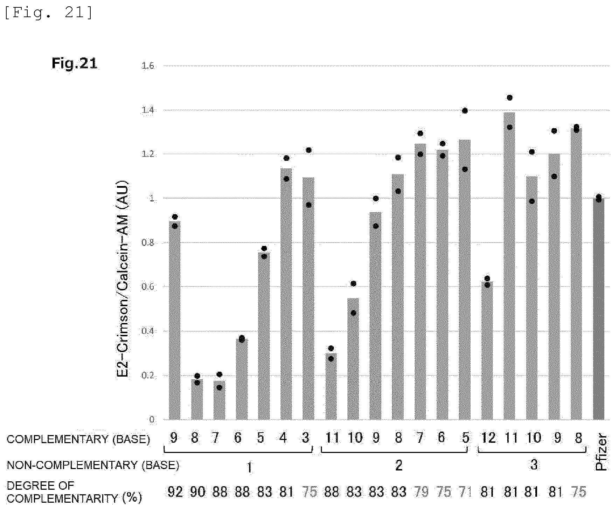

[FIG. 21] FIG. 21 is a graph showing the results of examining the expression level of the mRNA used in FIG. 19 in myoblast cell line C2C12. A ratio of an E2Crimson fluorescence measurement value to a Calcein fluorescence measurement value was defined as a relative expression level of E2Crimson protein in each mRNA. A plurality of samples were measured for each mRNA; the average value is shown in a bar graph, and the individual values of each sample are shown as black dots.

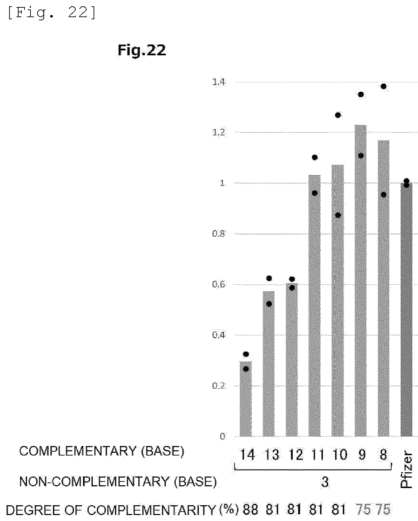

[FIG. 22] FIG. 22 is a graph showing the results of examining the expression level of the mRNA used in FIG. 20 in myoblast cell line C2C12. A ratio of an E2Crimson fluorescence measurement value to a Calcein fluorescence measurement value was defined as a relative expression level of E2Crimson protein in each mRNA. A plurality of samples were measured for each mRNA; the average value is shown in a bar graph, and the individual values of each sample are shown as black dots.

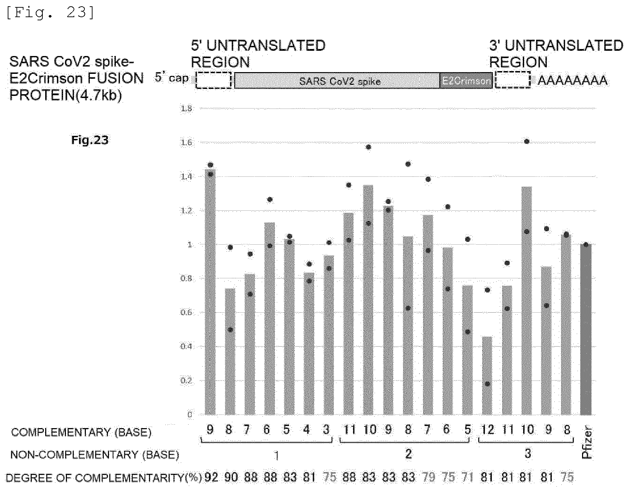

[FIG. 23] FIG. 23 is a graph showing the results of examining an expression level in 293 cells for mRNA having a Pfizer sequence as a 5' UTR, various sequences having partial complementarity with the 5' UTR as a 3' UTR, and SARS CoV2 Spike-E2Crimson fusion protein as ORF. In these 3' UTRs, the base is removed to form a non-complementary sequence. A ratio of an E2Crimson fluorescence measurement value to a Calcein fluorescence measurement value was defined as a relative expression level of E2Crimson protein in each mRNA. A plurality of samples were measured for each mRNA; the average value is shown in a bar graph, and the individual values of each sample are shown as black dots.

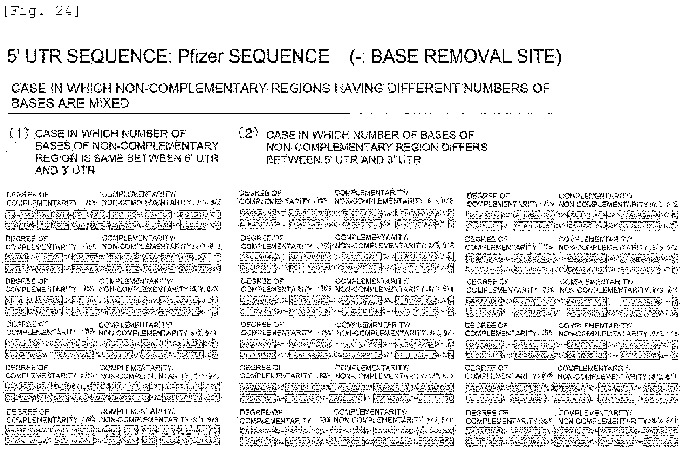

[FIG. 24] FIG. 24 illustrates base sequences of 5' UTR and 3' UTR of various mRNAs in which the 5' UTR is a Pfizer sequence and the 3' UTR is partially complementary to the 5' UTR. FIG. 24(1) illustrates a case in which the number of bases of the non-complementary region is the same in the 5' UTR and the 3' UTR, and FIG. 24(2) illustrates a case in which the number of bases of the non-complementary region differs between the 5' UTR and the 3' UTR. In each sequence, the upper row shows the 5' UTR sequence with the 5' end on the left side, and the lower row shows the 3' UTR sequence with the 5' end on the right side. A base indicated by "-" in the lower part indicates a base converted into a non-complementary sequence by removing the base in the complementary sequence. Description of ORF is omitted. Complementary bases between the 5' UTR and the 3' UTR are indicated by enclosures.

Description of Embodiments

Hereinafter, the present disclosure will be described while showing the best mode. Throughout the present specification, the expression of singular forms is to be understood as including the concept of plural forms unless otherwise stated. Therefore, the singular article (for example, "a", "an", "the", or the like in English) should be understood to include the concept of plural forms unless otherwise stated. In addition, the terms used in the present specification are to be understood as being used in the sense commonly used in the art unless otherwise stated. Therefore, unless defined otherwise, all technical terms and scientific terms used in the present specification have the same meaning as commonly understood by those skilled in the art to which the present disclosure pertains. In the case of conflict, the present specification (including definitions) will control.

Hereinafter, the definitions of the terms and/or basic technical concepts that are particularly used in the present specification will be described as appropriate.

As used herein, the term "about" refers to the indicated value plus or minus 10%.

As used herein, the term "untranslated region (UTR)" refers to a region that is not translated into a protein that is present before and after a coding region to be translated into a protein in a nucleic acid such as mRNA containing a portion encoding the protein. The UTR present on the 5' side of the nucleic acid is referred to as a 5' UTR, and the UTR present on the 3' side is referred to as a 3' UTR.

As used herein, the term (mutually) "complementary" or "complementarity" refers to a specific pair between adenine and thymine or uracil, or between guanine and cytosine or uracil (wobble base pair) in a base sequence of a nucleic acid. The "non-complementary region" refers to a region that is not complementary in the base sequence, and the "complementary region" refers to a region that is complementary in the base sequence. As used herein, a complementary region and a non-complementary region can be identified by one base unit. The phrase "lengths of all of the non-complementary regions are each 1 base" means that, when a complementary region and a non-complementary region are specified in a portion of a target nucleic acid, in a case in which there is a single non-complementary region, the length of the non-complementary region is 1 base, and in a case in which there are a plurality of non-complementary regions, the lengths of all of the plurality of non-complementary regions are 1 base. In addition, in the non-complementary region, either the 5' UTR or the 3' UTR may be 0 bases. In this case, in the present specification, the term "removal" is used (when viewed from the other side, it can also be referred to as "addition", but in the present specification, "removal" is adopted unless otherwise specified, and in a broad sense, "removal" is understood to include removal and addition in a narrow sense). In addition, in the non-complementary region, a case in which one or more non-complementary bases are present in the 5' UTR and the 3' UTR may be referred to as "substitution". Note that, when the number of bases on one side is different from the number of bases on the other side in the non-complementary region, the same number of portions corresponds to "substitution", and the smaller number of portions corresponds to "removal" with respect to the larger number of bases. For example,

in the case of 5'-NNNAGNNN

3'-NNNA0NNN

(N indicates a complementary strand and 0 indicates the absence of a base),

UA for A on the 5' side is a replacement of U in the complementary strand, and 0 for G on the 5' side corresponds to removal.

In addition,

in the case of 5'-NNNAGNNNU0NNN

3'-NNNA0NNNUGNNN,

the AG portion on the 5' side is the same as described above, and in the portion corresponding to U0, G can be considered as "addition", but in the present specification, in a broad sense, it may be described as "removal".

Note that, in the present specification, unless otherwise specified, removal, substitution, and addition are determined by comparing nucleic acids in order from the 5' side.

As used herein, the term "partially complementary" refers to being complementary over at least a portion (preferably, 2 or more bases) of a target base sequence. The number of bases of the complementary region is preferably 3 or more bases and 11 or less bases.

As used herein, the term "nucleic acid construct" refers to a construct at least partially composed of nucleic acids, and typically refers to a (non-natural) nucleic acid molecule (for example, a recombinant nucleic acid) resulting from the use of recombinant DNA technology. Typically, a nucleic acid construct is a single-stranded or double-stranded nucleic acid molecule that is modified to contain segments of nucleic acid sequence that are combined and arranged in a manner not found in nature. A nucleic acid construct may be a "vector" (for example, a plasmid, an rAAV vector genome, an expression vector, or the like), that is, a nucleic acid molecule designed to deliver exogenously generated DNA to a host cell.

As used herein, the term "degree of complementarity (%)" refers to a percentage of identity bases of the single-stranded nucleic acid with respect to the base sequence of the complementary sequence of the reference sequence. The degree of complementarity is calculated based on the length of the reference sequence. When the complementary sequence of the reference sequence and the base sequence of the single-stranded nucleic acid are perfectly matched, the degree of complementarity is 100%. In the embodiment in which the non-complementary region is removed in the present disclosure, the percentage of identity is calculated assuming that the non-complementary region is present. As used herein, the "protein", "polypeptide", and "peptide" are used in the same meaning, and refer to an amino acid polymer of any length. The polymer may be linear, branched, or cyclic. An amino acid may be a natural, non-natural, or modified amino acid. The term also encompasses a natural or artificially modified polymer. Examples of such a modification include disulfide bond formation, glycosylation, lipidation, acetylation, phosphorylation, and any other manipulation or modification (for example, conjugation with a labeling component). The amino acid may be represented in the present disclosure by commonly known three-letter symbols or one-letter symbols recommended by the IUPAC-IUB Biochemical Nomenclature Commission. Similarly, the nucleotide may be represented by commonly accepted single-letter codes. Note that, in the present specification, since the display of nucleotides follows the rules of the sequence listing, T may be displayed even in the case of RNA, but those skilled in the art understand that T means U when T is displayed for RNA.

As used herein, the "polynucleotide" and "nucleic acid" are used to have the same meaning, and refer to a polymer of nucleotides of any length. Examples of the nucleic acid include DNA, RNA, cDNA, mRNA, rRNA, tRNA, microRNA (miRNA), and lncRNA. The term also includes a "polynucleotide derivative". The "polynucleotide derivative" refers to a polynucleotide containing a nucleotide derivative or having an unusual bond between nucleotides. The "nucleotide derivative" refers to a nucleotide having a structure different from a normal nucleotide used in natural DNA or RNA, and examples thereof include a locked nucleic acid (LNA), an ethylene nucleic acid such as a 2'-O,4'-C-ethylene bridged nucleic acid (ENA), other bridged nucleic acids (BNAs), a hexitol nucleic acid (HNA), an amido-bridged nucleic acid (AmNA), a morpholino nucleic acid, tricyclo-DNA (tcDNA), a polyether nucleic acid (see, for example, US 5,908,845), and a cyclohexene nucleic acid (CeNA). Examples of the unusual bond between nucleotides include a bond between oligonucleotides in which a phosphate diester bond is converted into a phosphorothioate bond, a bond between oligonucleotides in which a phosphate diester bond is converted into an N3'-P5' phosphoramidate bond, and a bond between oligonucleotides in which ribose and a phosphate diester bond are converted to peptide nucleic acid bonds.

As used herein, the "gene" refers to a nucleic acid moiety that performs a certain biological function. The biological function includes coding for polypeptides or proteins, coding for protein non-coding functional RNAs (rRNA, tRNA, microRNA (miRNA), lncRNA, and the like), regulating the production of polypeptides, proteins, or non-coding functional RNAs, specifically binding to specific proteins, and regulating cleavage or replication of nucleic acids.

As used herein, the "kit" refers to a unit generally providing portions to be provided (for example, a nucleic acid construct, instructions, and the like) that are usually divided into two or more portions. The form of the kit is preferred when it is intended to provide a composition that should not be provided in a mixed state for stability or the like, but is preferably mixed immediately before use. Such a kit preferably includes an instruction or manual describing how to use the provided portions or how a reagent should be processed. When the kit is used in the present specification as a reagent kit, the kit generally includes an instruction or the like describing how to use a nucleic acid construct and the like.

As used herein, the "instruction" is a document with an explanation of the method of use of the present disclosure for a user. The instruction includes language directing how to use the nucleic acid construct of the present disclosure. The instruction may be usually provided in, but is not limited to, a paper medium, and for example, the instruction may also be provided in a form such as an electronic medium (for example, a web site or an e-mail provided on the Internet).

As used herein, the "change in (Gibbs) free energy ($\Delta$G)" is thermodynamically or statistically described by the following Equations (1) and (2).

$$\Delta G \ = \ \Delta H \ - \ T\Delta S \quad (1)$$

$$\Delta G \ = \ \Delta G° \ + \ RT\ln K \quad (2)$$

Here, K is an equilibrium constant, and $\Delta$G° is the change in Gibbs free energy in a standard state (1 atm, 25°C). When the equilibrium constant of the nucleic acid is considered, it can be described as an equilibrium reaction in which two strands A and B (for example, 3' UTR and 5' UTR) associate at a ratio of 1:1 (Equation (3)).

$$A \ + \ B \ \Leftrightarrow \ AB \quad (3)$$

When the state molar fraction of the double strand is $\alpha$ and the total concentration of the nucleic acid is C, the concentrations [A] and [B] of A and B when completely dissociated are C/2, and thus, the equilibrium constant can be represented by the following Equation (4).

$$K \ = \ 2\alpha/((1 \ - \ \alpha)^2 \ \times \ C) \quad (4)$$

When the nucleic acid is in an equilibrium state deviated by 50%,

$$\alpha \ = \ 1/2, \ \Delta G \ = \ 0 \quad (5);$$

thus, by substituting Equations (4) and (5) into Equation (2), the following Equation (6) is obtained.

$$\Delta G° \ = \ -RTm\ln (4/C) \quad (6)$$

By substituting Equation (6) into Equation (1), the following Equation (7) is obtained.

$$1/Tm \ = \ (R/\Delta H°)\ln(Ct/4) \ + \ \Delta S°/\Delta H° \quad (7)$$

When the measured values are plotted as two functions of 1/Tm and ln(C/4), the change in enthalpy $\Delta$H° and the change in entropy $\Delta$S° in the standard state are obtained from the intercept and the slope.

(Preferred embodiments)

Preferred embodiments of the present disclosure will be described below. It is understood that the embodiments provided below are provided for a better understanding of the present disclosure, and that the scope of the present disclosure should not be limited to the following description. Therefore, it is apparent that those skilled in the art can appropriately make modifications within the scope of the present disclosure in view of the description in the present specification. In addition, it is also understood that the following embodiments may be used alone or in combination.

In one aspect, the present disclosure provides a nucleic acid construct including a 5' untranslated region (UTR) and a 3' UTR that are at least partially complementary to each other, in which at least one of UTRs includes a non-complementary region and a complementary region with respect to the other UTR, and when lengths of all of the non-complementary regions are each 1 base, a degree of complementarity of the 3' UTR to the 5' UTR is greater than 75%. The nucleic acid construct of the present disclosure can regulate protein expression. Preferably, the nucleic acid construct of the present disclosure can increase protein expression.

In some embodiments, each of at least 1, at least 2, at least 3, at least 4, at least 5, at least 6, at least 7, at least 8, at least 9, at least 10, at least 15, and at least 20 non-complementary regions may be 2 bases or 3 bases in length. In a preferred embodiment, the lengths of all of the non-complementary regions are 2 bases or 3 bases.

In another embodiment, the lengths of all of the non-complementary regions may each be 1 base, and in this case, a degree of complementarity of the 3' UTR to the 5' UTR may be greater than 75%, for example, greater than 75% and 90% or less, greater than 75% and 89% or less, 80% or more and 90% or less, or 81% or more and 89% or less.

In some embodiments, at least 1, at least 2, at least 3, at least 4, at least 5, at least 6, at least 7, at least 8, at least 9, at least 10, at least 15, or at least 20 complementary regions may be 5 or more bases, for example, 5 bases, 6 bases, 7 bases, 8 bases, 9 bases, 10 bases, 11 bases, 12 bases, 13 bases, 15 bases, or 20 bases. In a preferred embodiment, at least 1, at least 2, at least 3, at least 4, at least 5, at least 6, at least 7, at least 8, at least 9, at least 10, at least 15, or at least 20 complementary regions may be 5 to 11 bases in length. The lengths of the complementary regions may be all the same or different, and are preferably all the same.

In some embodiments, a length of the non-complementary region is 2 bases, and lengths of the complementary regions may each independently be 5 to 7 bases.

In some embodiments, a length of the non-complementary region is 3 bases, and lengths of the complementary regions may each independently be 8 to 11 bases.

In one embodiment, the present disclosure provides a nucleic acid construct including a 5' untranslated region (UTR) and a 3' UTR that are at least partially complementary to each other, in which at least one of UTRs includes a non-complementary region (2) and a complementary region (3) with respect to the other UTR, and the non-complementary region and the complementary region are alternately present.

In one embodiment, a region other than the alternately present regions (also referred to as a residual region (4)) is 1 or more bases and less than or equal to a base length of the complementary region.

In one embodiment, when the non-complementary region (2) is 1 base, the complementary region (3) is 3 to 6 bases in length in a case of base substitution and 3 to 9 bases in length in a case of base removal.

In one embodiment, when the non-complementary region (2) is 2 bases, the complementary region (3) is 5 to 7 bases in length in a case of base substitution and 5 to 11 bases in length in a case of base removal.

In one embodiment, when the complementary region (2) is 3 bases, the complementary region (3) is 8 to 9 bases in length in a case of base substitution and 8 to 11 bases in length in a case of base removal.

In one embodiment, the non-complementary region (2) is usually 0 to 3 bases, and preferably 2 bases or 3 bases.

As described above, the appropriate base length can vary by substitution and removal, but those skilled in the art can appropriately design the base length based on the description of the present specification.

In one embodiment, the non-complementary region (2) may have two or more different lengths in the untranslated region. In this case, it is advantageous that at least one of the preferred base lengths is satisfied, and it is advantageous that all of the preferred base lengths are satisfied.

In one embodiment, the non-complementary region (2) may have a mixture of substitution and removal in the untranslated region. In this case, in this case, it is advantageous that at least one of the preferred base lengths is satisfied, and it is advantageous that all of the preferred base lengths are satisfied.

In some embodiments, when the lengths of all of the non-complementary regions are all 2 bases or 3 bases, the degree of complementarity may be at least 60% or more, at least 65% or more, at least 70% or more, at least 75% or more, at least 80% or more, at least 85% or more, or at least 90% or more. In a specific embodiment, when the lengths of all of the non-complementary regions are all 2 bases or 3 bases, the degree of complementarity may be greater than 75%, for example, greater than 75% and 90% or less, greater than 75% and 89% or less, 80% or more and 90% or less, or 81% or more and 89% or less.

The lengths of the complementary region and the non-complementary region can be appropriately determined according to a desired degree of complementarity, and typically, the lengths of the non-complementary regions may each independently be 1 to 3 bases, and the lengths of the complementary regions may each independently be 3 to 11 bases.

In a specific embodiment, when the non-complementary regions are all 1 base, the lengths of the complementary regions may each independently be 3 to 7 bases.

In a specific embodiment, when the non-complementary regions are all 2 bases, the lengths of the complementary regions may each independently be 5 to 7 bases.

In a specific embodiment, when the non-complementary regions are all 3 bases, the lengths of the complementary regions may each independently be 8 to 11 bases.

In a specific embodiment, a non-complementary region may be removed in either the 5' UTR or the 3' UTR.

In some embodiments, the UTR can be selected from a UTR of GAPDH, HSD17B4, PSMB3, RPL31, RPL32, RPL35, RPL21, Albumin7, LDHB, ACAT2, ATP5A1, Ndufa4, Mp68, NOSIP, SLC7A3, TUBB4B, UBQLN2, mRPL35A, mRPL21, AIG1, COX6C, α-globin, β-globin, RPS8, TOP, MCP-1, RPL12s.c., Ang-2, HSP70, H3.3., Galectin-9, GADD34, EDN1, HSP70m5, E-selectin, ICAM-1, IL-6, or vWF. Other examples of UTR are disclosed in JP 2021-501572 A, JP 2015-517803 A, and JP 2022-164843 A.

In another aspect, a nucleic acid construct of the present disclosure may be provided as a kit.

In a further aspect, the present disclosure provides a method of producing a nucleic acid construct, the nucleic acid construct including a 5' untranslated region (UTR) and a 3' UTR that are at least partially complementary to each other, in which at least one of UTRs includes a non-complementary region and a complementary region to the other UTR, the method including: a step of designing a plurality of candidate nucleic acid constructs; a step of calculating a change in free energy ($\Delta$G) of a secondary structure of the plurality of designed candidate nucleic acid constructs; a step of selecting a candidate nucleic acid construct of which a change in free energy is a predetermined value or a range thereof (for example, a rate of increase of 0% to 60% with respect to an average value of a maximum value ($\Delta G_{max}$) and a minimum value ($\Delta G_{min}$)); and a step of optionally measuring an expression level of the selected candidate nucleic acid construct in cells.

In some embodiments, the change in free energy ($\Delta$G (kcal/mol)) can be calculated by a nucleic acid secondary structure prediction program, for example, mfold (GCG Software), IPknot, CentroidFold (https://www.ncrna.org/), ViennaRNA (http://rna.tbi.univie.ac.at/), RNALOSS (P Clote, RNALOSS: a web server for RNA locally optimal secondary structures, Nucleic Acids Res. 2005 Jul 1; 33: W600-4.), or RNA Secondary structure prediction (http://www.genebee.msu.su/services/rna2_reduced.html).

The predetermined value of the change in free energy or the range thereof can be appropriately determined by those skilled in the art with reference to Examples 6 and 7. Specifically, by preparing a nucleic acid construct having various degrees of complementarity and correlating the protein expression level by the nucleic acid construct with the change in free energy, a predetermined value of the change in free energy at which the expression level is maximized or a range thereof can be determined.

In some embodiments, the free energy of the secondary structure of the nucleic acid construct of the present disclosure may be

$$((\Delta G_{max} + \Delta G_{min})/2) \times t. \quad \text{Here, } t = 0.9 \text{ to } 1.7,$$

preferably t = 1 to 1.6, and most preferably t = 1.1 to 1.5.

In some embodiments, the change in free energy may be a change in free energy of the interaction between the 3' UTR and the 5' UTR in the nucleic acid construct.

In some embodiments, the maximum value ($\Delta G_{max}$) of the change in free energy is a change in free energy of an interaction between all bases of a 3' UTR or 5' UTR and a 5' UTR or 3' UTR sequence in which a mismatched substitution is introduced into its complementary strand (that is, a sequence having a degree of complementarity of 0%), and the minimum value ($\Delta G_{min}$) of the change in free energy may be a change in free energy of an interaction between a 5' UTR and a 3' UTR of a sequence having 100% 5'-3' UTR complementarity.

In a specific embodiment, the rate of increase with respect to the average value may be 0% to 60%, and preferably 10% to 50%.

The present disclosure may be applied in an application field (for example, a medicine) of nucleic acid technology. For example, mRNA which is a nucleic acid construct of the present disclosure can also be used as a drug substance of an mRNA medicine. In addition, the plasmid DNA which is the nucleic acid construct of the present disclosure can be used to create a cell bank used for the production of an mRNA drug substance. In addition, the linear template DNA which is the nucleic acid construct of the present disclosure can also be used as a raw material during the production process of the mRNA drug substance.

As used herein, "or" is used when "at least one or more" of the items listed in the text can be employed. The same applies to "or". When explicitly described in the present specification as "within the range" of "two values", the range also includes the two values themselves.

Reference literatures such as scientific literatures, patents, and patent applications cited in the present specification are incorporated herein by reference to the same extent that the entirety of each document is specifically described.

The present disclosure has been described above with reference to preferred embodiments for easy understanding. Hereinafter, the present disclosure will be described based on examples, but the above description and the following examples are provided for the purpose of illustration only and not for the purpose of limiting the present disclosure. Accordingly, the scope of the present disclosure is not limited to the embodiments or examples that are specifically described in the present specification, but is limited only by the claims.

Examples

For reagents, the specific products described in the examples were used. However, the reagent can be substituted with an equivalent product from another manufacturer (Sigma-Aldrich, Wako Pure Chemical, NACALAI TESQUE, INC., R&D Systems, USCN Life Science INC, or the like).

(Molecular biology experimental manipulation)

Common DNA, RNA, and genetic recombination experimental manipulations were performed according to the standard protocol (Sambrook, J., et al., Molecular Cloning: A Laboratory Manual. Cold Spring Harbor Laboratory Press, Cold Spring Harbor, New York (1989)).

(Production examples)

(Purification of DNA fragment using low-melting-point agarose gel)

DNA fragments were separated by electrophoresis at a voltage of 100 V for 1 hour using a 0.7% low-melting-point agarose gel prepared using 2-hydroxyethyl agarose (Sigma-Aldrich) and $1 \times$ TAE buffer (NACALAI TESQUE, INC.). The gel after electrophoresis was stained with $1 \times$ TAE buffer containing GelRed nucleic acid gel stain (FUJIFILM Wako Pure Chemical Corporation) for 30 minutes, and visualized with a long-wavelength ultraviolet ray (366 nm) to recover a target DNA fragment from agarose gel. The agarose gel containing the DNA fragments was dissolved by incubating at 63°C for 5 minutes and further incubating at 60°C for 10 minutes in the presence of Thermostable β-Agarase (NIPPON GENE CO., LTD.), and then TE saturated phenol (NACALAI TESQUE, INC.) was added and sufficiently mixed. A phenol phase and an aqueous phase were separated by centrifugation ($20,000 \times$ g, 10 minutes), and the aqueous phase was recovered in a new tube. The recovered aqueous phase was separated into a 1-butanol phase and an aqueous phase by centrifugation ($20,000 \times$ g, 10 minutes) after addition of 1-butanol and sufficient mixing, and a series of operations of removing the separated 1-butanol phase was repeated three times to remove phenol and reduce the volume of the aqueous phase. To the aqueous phase, a 3 M potassium acetate-acetic acid buffer solution (pH 5.2) and ethanol were added and sufficiently mixed, and then centrifuged ($20,000 \times$ g, 10 minutes) to precipitate DNA fragments. The precipitated DNA fragments were washed with 70% ethanol and then dissolved in TE buffer (NACALAI TESQUE, INC.).

(Annealing of synthetic oligonucleotide)

Two types of 100 μM synthetic oligonucleotides mixed at 5 μl each were incubated at 99°C for 10 seconds using TaKaRa PCR Thermal Cycler Dice Touch (Takara Bio Inc.), and then gradually cooled to 40°C for 90 minutes to form 50 μM double-stranded oligonucleotides. The double-stranded oligonucleotides were diluted to 0.5 μM with distilled water.

(Ligation, E. coli transformation, and plasmid preparation)

1 μl of a plasmid solution treated with 1 to 20 ng/μl of a restriction enzyme, 1 μl of a 0.1 to 20 ng/μl DNA fragment solution or 1 μl of a 0.5 μM double-stranded oligonucleotide solution, and 2 μl of DNA Ligation Kit <Mighty Mix> (Takara Bio Inc.) were mixed, and the mixture was incubated at 16°C for 1 to 4 hours to perform a ligation reaction. 1 μl of the reaction solution and 10 μl of E. coli JM109 competent cells (Takara Bio Inc.) were mixed, and the mixture was allowed to stand on ice for 1 hour, and then incubated in a hot water bath at 42°C for 1 minute. The E. coli after incubation was allowed to stand on ice for 2 minutes, 50 μl of the SOC medium was added, and E. coli was cultured by a small rotary culture machine RT-50 (TIETECH Co., Ltd.) at a rotation speed of 30 rpm and 37°C for 1 hour. E. coli after culture was smeared on an LB medium agar plate containing 10 μg/ml of carbenicillin and cultured overnight at 37°C. The E. coli colonies formed on the agar plate were inoculated into 2 ml of an LB liquid medium containing 10 μg/ml of carbenicillin, and shaken overnight at a rotation speed of 220 rpm and 37°C using a medium constant temperature shaking culture machine BR-53FP (TIETECH Co., Ltd.). E. coli after culture was recovered by centrifugation ($20,000 \times$ g, 1 minute), and a plasmid was prepared by QIAprep Spin Miniprep Kit and a nucleic acid extraction and purification apparatus QIAcube (QIAGEN N.V.) according to the manufacturer's instruction manual.

(Construction of plasmid as template for RNA synthesis)

A DNA fragment composed of a T7 promoter sequence, a Kozak sequence, a stop codon, and a polyA continuous sequence having a length of 101 bases was amplified by PCR using primers Common-F and Common-R (SEQ ID NOs: 3 and 4) and KOD-Plus-Ver. 2, with a double-stranded product obtained by annealing synthetic oligonucleotides set forth in SEQ ID NOs: 1 and 2 partially complementary at their 3' ends, and subjecting the single-stranded portions to polymerase extension reaction in a direction from 5' to 3' using KOD-Plus-Ver. 2 (Toyobo Co., Ltd.), as a template. The DNA fragment has a BsaI site for inserting a 5' UTR between the T7 promoter sequence and the Kozak sequence, a BspQI site for inserting a translation region (ORF) between the Kozak sequence and the stop codon, a PaqCI site for inserting a 3' UTR between the stop codon and the polyA sequence, and a BsmBI site for linearization of the template DNA downstream of the polyA continuous sequence. The amplified DNA fragment was purified by MinElute PCR Purification Kit (QIAGEN N.V.), and then inserted into an XcmI site of pBR322_ΔtypeIIS plasmid to construct pT7_TL_pA100 plasmid (SEQ ID NO: 135).

A DNA fragment encoding fluorescent protein E2Crimson was amplified by PCR using primers Common-F and Common-R (SEQ ID NOs: 3 and 4) and KOD-Plus-Ver. 2, with a double-stranded product obtained by annealing each of synthetic oligonucleotides of SEQ ID NOs: 5 and 6, SEQ ID NOs: 7 and 8, and SEQ ID NOs: 9 and 10 partially complementary at their 3' ends, and subjecting these single-stranded portions to polymerase extension reaction in a direction from 5' to 3' using KOD-Plus-Ver. 2 (Toyobo Co., Ltd.), as a template. The amplified DNA fragment was inserted into an XcmI site of pBR322_ΔtypeIIS plasmid to construct pBR_E2Crimson plasmid. pT7_TL_E2Crimson_pA100 plasmid was constructed by inserting the E2CrimsonDNA fragment obtained by BspQI treatment of pBR_E2Crimson into a PaqCI site of pT7_TL_pA100. A DNA fragment encoding SARS CoV2 Spike protein was a sequence described in messenger RNA encoding the full-length SARS-CoV-2 spike glycoprotein Sept. 2020 document 11889; the material of the 19th Health Sciences Council of the Subcommittee on Immunization and Vaccination (https://www.mhlw.go.jp/stf/shingi2/0000192554_00004.html). The DNA fragment was inserted upstream of the region encoding E2Crimson in the pT7_TL_E2Crimson_pA100 plasmid to construct pT7_TL_Spk_E2Crimson_pA100 plasmid.

The Pfizer 5' UTR sequence was obtained by annealing synthetic oligonucleotides set forth in SEQ ID NOs: 13 and 14. The DNA fragment was inserted into a BsaI site of pT7_TL_E2Crimson_pA100 or pT7_TL_Spk_E2Crimson_pA100 to construct pT7_TL_5Pf_E2Crimson_pA100 plasmid or pT7_TL_5Pf_Spk_E2Crimson_pA100 plasmid. The Pfizer 3' UTR sequence was amplified by PCR using primers Common-F and Common-R (SEQ ID NOs: 3 and 4) and KOD-Plus-Ver. 2, with a double-stranded product obtained by annealing synthetic oligonucleotides set forth in SEQ ID NOs: 11 and 12 partially complementary at their 3' ends, and subjecting the single-stranded portions to polymerase extension reaction in a direction from 5' to 3' using KOD-Plus-Ver. 2 (Toyobo Co., Ltd.), as a template. pBR-3Pf plasmid was constructed by cloning the PCR fragment into an XcmI site of the pBR322_ΔtypeIIS plasmid. The Pfizer 3' UTR sequence obtained by BsaI-treating the pBR-3Pf plasmid was inserted into a PaqCI site of pT7_TL_5Pf_E2Crimson_pA100 or pT7_TL_5Pf_Spk_E2Crimson_pA100 to construct pT7_TLpA_5Pf_E2Crimson_3Pf_pA100 (SEQ ID NO: 136) or pT7_TL_5Pf_Spk_E2Crimson_3Pf_pA100 (SEQ ID NO: 300).

The 5' UTR sequence of the GAPDH gene was obtained by annealing synthetic oligonucleotides set forth in SEQ ID NOs: 15 and 16. The DNA fragment was inserted into a BsaI site of pT7_TL_E2Crimson_pA100 to construct pT7_TL_5GAP_E2Crim_pA100 plasmid. A 3' UTR sequence partially complementary to the 5' UTR sequence of the GAPDH gene was obtained by annealing synthetic oligonucleotides described below. The DNA fragment was inserted into a PaqCI site of pT7_TL_5GAP_E2Crim_pA100 to construct pT7_TL_5GAP_E2Crim_3UTR_pA100 plasmid. The sequence numbers of the synthetic oligonucleotides and the combination of the constructed plasmids are shown in Table 1.

[Table 1]

Table 1

| Synthetic oligonucleotide SEQ ID NO | Plasmid SEQ ID NO | Plasmid name |
| --- | --- | --- |
| 17, 18 | 137 | pT7_TL_5GAP_E2Crim_3UTR-100_pA100 |
| 19, 20 | 138 | pT7_TL_5GAP_E2Crim_3UTR94-2_pA100 |
| 21, 22 | 139 | pT7_TL_5GAP_E2Crim_3UTR75-4_pA100 |
| 23, 24 | 140 | pT7_TL_5GAP_E2Crim_3UTR75-5_pA100 |
| 25, 26 | 141 | pT7_TL_5GAP_E2Crim_3UTR75-6_pA100 |
| 27, 28 | 142 | pT7_TL_5GAP_E2Crim_3UTR75-7_pA100 |
| 29, 30 | 143 | pT7_TL_5GAP_E2Crim_3UTR-10-1_pA100 |
| 31, 32 | 144 | pT7_TL_5GAP_E2Crim_3UTR-9-1_pA100 |
| 33, 34 | 145 | pT7_TL_5GAP_E2Crim_3UTR-8-1_pA100 |

(continued)

| Synthetic oligonucleotide SEQ ID NO | Plasmid SEQ ID NO | Plasmid name |
|---|---|---|
| 35, 36 | 146 | pT7_TL_5GAP_E2Crim_3UTR-7-1_pA100 |
| 37, 38 | 147 | pT7_TL_5GAP_E2Crim_3UTR-6-1_pA100 |
| 39, 40 | 148 | pT7_TL_5GAP_E2Crim_3UTR-5-1_pA100 |
| 41, 42 | 149 | pT7_TL_5GAP_E2Crim_3UTR-4-1_pA100 |
| 43, 44 | 150 | pT7_TL_5GAP_E2Crim_3UTR-3-1_pA100 |
| 45, 46 | 151 | pT7_TL_5GAP_E2Crim_3UTR-13-2_pA100 |
| 47, 48 | 152 | pT7_TL_5GAP_E2Crim_3UTR-10-2_pA100 |
| 49, 50 | 153 | pT7_TL_5GAP_E2Crim_3UTR-9-2_pA100 |
| 51, 52 | 154 | pT7_TL_5GAP_E2Crim_3UTR-8-2_pA100 |
| 53, 54 | 155 | pT7_TL_5GAP_E2Crim_3UTR-6-2_pA100 |
| 55, 56 | 156 | pT7_TL_5GAP_E2Crim_3UTR-20-3_pA100 |
| 57, 58 | 157 | pT7_TL_5GAP_E2Crim_3UTR-14-3_pA100 |
| 59, 60 | 158 | pT7_TL_5GAP_E2Crim_3UTR-11-3_pA100 |

A 3' UTR sequence partially complementary to the Pfizer 5' UTR sequence was obtained by annealing synthetic oligonucleotides described below. The DNA fragment was inserted into a PaqCI site of pT7_TL_5Pf_E2Crimson_pA100 to construct pT7_TL_5Pf_E2Crim_3UTR-100_pA100 plasmid. The sequence numbers of the synthetic oligonucleotides and the combination of the constructed plasmids are described below.

[Table 2-1]

Table 2-1

| Synthetic oligonucleotide SEQ ID NO | Plasmid SEQ ID NO | Plasmid name |
|---|---|---|
| 61, 62 | 159 | pT7_TL_5Pf_E2Crim_3UTR-100_pA100 |
| 63, 64 | 160 | pT7_TL_5Pf_E2Crim_3UTR-9-1_pA100 |
| 65, 66 | 161 | pT7_TL_5Pf_E2Crim_3UTR-8-1_pA100 |
| 67, 68 | 162 | pT7_TL_5Pf_E2Crim_3UTR-7-1_pA100 |
| 69, 70 | 163 | pT7_TL_5Pf_E2Crim_3UTR-6-1_pA100 |
| 71, 72 | 164 | pT7_TL_5Pf_E2Crim_3UTR-5-1_pA100 |
| 73, 74 | 165 | pT7_TL_5Pf_E2Crim_3UTR-4-1_pA100 |
| 75, 76 | 166 | pT7_TL_5Pf_E2Crim_3UTR-3-1_pA100 |
| 77, 78 | 167 | pT7_TL_5Pf_E2Crim_3UTR-11-2_pA100 |
| 79, 80 | 168 | pT7_TL_5Pf_E2Crim_3UTR-10-2_pA100 |
| 81, 82 | 169 | pT7_TL_5Pf_E2Crim_3UTR-9-2_pA100 |
| 83, 84 | 170 | pT7_TL_5Pf_E2Crim_3UTR-8-2_pA100 |
| 85, 86 | 171 | pT7_TL_5Pf_E2Crim_3UTR-7-2_pA100 |
| 87, 88 | 172 | pT7_TL_5Pf_E2Crim_3UTR-6-2_pA100 |
| 89, 90 | 173 | pT7_TL_5Pf_E2Crim_3UTR-5-2_pA100 |
| 91, 92 | 174 | pT7_TL_5Pf_E2Crim_3UTR-12-3_pA100 |
| 93, 94 | 175 | pT7_TL_5PC_E2Crim_3UTR-10-3_pA100 |

(continued)

| Synthetic oligonucleotide SEQ ID NO | Plasmid SEQ ID NO | Plasmid name |
|---|---|---|
| 95, 96 | 176 | pT7_TL_5Pf_E2Crim_3UTR-9-3_pA100 |
| 97, 98 | 177 | pT7_TL_5Pf_E2Crim_3UTR-8-3_pA100 |
| 216, 217 | 258 | pT7_TL_5Pf_E2Crim_3UTR_M1-9-1_pA100 |
| 218, 219 | 259 | pT7_TL_5Pf_E2Crim_3UTR_M2-8-1_pA100 |
| 220, 221 | 260 | pT7_TL_5Pf_E2Crim_3UTR_M3-7-1_pA100 |
| 222, 223 | 261 | pT7_TL_5Pf_E2Crim_3UTR_M4-6-1_pA100 |
| 224, 225 | 262 | pT7_TL_5Pf_E2Crim_3UTR_M5-5-1_pA100 |
| 226, 227 | 263 | pT7_TL_5Pf_E2Crim_3UTR_M6-4-1_pA100 |
| 228, 229 | 264 | pT7_TL_5Pf_E2Crim_3UTR_M7-3-1_pA100 |
| 230, 231 | 265 | pT7_TL_5Pf_E2Crim_3UTR_M8-11-2_pA100 |
| 232, 233 | 266 | pT7_TL_5Pf_E2Crim_3UTR_M9-10-2_pA100 |

[Table 2-2]

Table 2-2

| | | |
|---|---|---|
| 234, 235 | 267 | pT7_TL_5Pf_E2Crim_3UTR_M10-9-2_pA100 |
| 236, 237 | 268 | pT7_TL_5Pf_E2Crim_3UTR_M11-8-2_pA100 |
| 238, 239 | 269 | pT7_TL_5Pf_E2Crim_3UTR_M12-7-2_pA100 |
| 240, 241 | 270 | pT7_TL_5Pf_E2Crim_3UTR_M13-6-2_pA100 |
| 242, 243 | 271 | pT7_TL_5Pf_E2Crim_3UTR_M14-5-2_pA100 |
| 244, 245 | 272 | pT7_TL_5Pf_E2Crim_3UTR_M15-12-3_pA100 |
| 246, 247 | 273 | pT7_TL_5Pf_E2Crim_3UTR_M16-11-3_pA100 |
| 248, 249 | 274 | pT7_TL_5Pf_E2Crim_3UTR_M17-10-3_pA100 |
| 250, 251 | 275 | pT7_TL_5Pf_E2Crim_3UTR_M18-9-3_pA100 |
| 252, 253 | 276 | pT7_TL_5Pf_E2Crim_3UTR_M19-8-3_pA100 |
| 254, 255 | 277 | pT7_TL_5Pf_E2Crim_3UTH_M20-14-3_pA100 |
| 256, 257 | 278 | pT7_TL_5Pf_E2Crim_3UTR_M21-13-3_pA100 |
| 216, 217 | 279 | pT7_TL_5Pf_Spk_E2Crim_3UTR_M1-9-1_pA100 |
| 218, 219 | 280 | pT7_TL_5Pf_Spk_E2Crim_3UTR_M2-8-1_pA100 |
| 220, 221 | 281 | pT7_TL_5Pf_Spk_E2Crim_3UTR_M3-7-1_pA100 |
| 222, 223 | 282 | pT7_TL_5Pf_Spk_E2Crim_3UTR_M4-6-1_pA100 |
| 224, 225 | 283 | pT7_TL_5Pf_Spk_E2Crim_3UTR_M5-5-1_pA100 |
| 226, 227 | 284 | pT7_TL_5Pf_Spk_E2Crim_3UTR_M6-4-1_pA100 |
| 228, 229 | 285 | pT7_TL_5Pf_Spk_E2Crim_3UTR_M17-3-1_pA100 |
| 230, 231 | 286 | pT7_TL_5Pf_Spk_E2Crim_3UTR_M8-11-2_pA100 |
| 232, 233 | 287 | pT7_TL_5Pf_Spk_E2Crim_3UTR_M9-10-2_pA100 |
| 234, 235 | 288 | pT7_TL_5Pf_Spk_E2Crim_3UTR_M10-9-2_pA100 |
| 236, 237 | 289 | pT7_TL_5Pf_Spk_E2Crim_3UTR_M11-8-2_pA100 |
| 238, 239 | 290 | pT7_TL_5Pf_Spk_E2Crim_3UTR_M12-7-2_pA100 |

(continued)

| | | |
|---|---|---|
| 240, 241 | 291 | pT7_TL_5Pf_Spk_E2Crim_3UTR_M13-6-2_pA100 |
| 242, 243 | 292 | pT7_TL_5Pf_Spk_E2Crim_3UTR_M14-5-2_pA100 |
| 244, 245 | 293 | pT7_TL_5Pf_Spk_E2Crim_3UTR_M15-12-3_pA100 |
| 246, 247 | 294 | pT7_TL_5Pf_Spk_E2Crim_3CTR_M16-11-3_pA100 |
| 248, 249 | 295 | pT7_TL_5Pf_Spk_E2Crim_3UTR_M17-10-3_pA100 |
| 250, 251 | 296 | pT7_TL_5Pf_Spk_E2Crim_3UTR_M18-9-3_pA100 |
| 252, 253 | 297 | pT7_TL_5Pf_Spk_E2Crim_3UTR_M19-8-3_pA100 |
| 254, 255 | 298 | pT7_TL_5Pf_Spk_E2Crim_3UTR_M20-14-3_pA100 |
| 256, 257 | 299 | pT7_TL_5Pf_Spk_E2Crim_3UTR_M21-13-3_pA100 |
| 216, 217 | 258 | pT7_TL_5Pf_E2Crim_3UTR_M1-9-1_pA100 |
| 218, 219 | 259 | pT7_TL_5Pf_E2Crim_3UTR_M2-8-1_pA100 |

[Table 2-3]

Table 2-3

| | | |
|---|---|---|
| 220, 221 | 260 | pT7_TL_5Pf_E2Crim_3UTR_M3-7-1_pA100 |
| 222, 223 | 261 | pT7_TL_5Pf_E2Crim_3UTR_M4-6-1_pA100 |
| 224, 225 | 262 | pT7_TL_5Pf_E2Crim_3UTR_M5-5-1_pA100 |
| 226, 227 | 263 | pT7_TL_5Pf_E2Crim_3UTR_M6-4-1_pA100 |
| 228, 229 | 264 | pT7_TL_5Pf_E2Crim_3UTR_M7-3-1_pA100 |
| 230, 231 | 265 | pT7_TL_5Pf_E2Crim_3UTR_M8-11-2_pA100 |
| 232, 233 | 266 | pT7_TL_5Pf_E2Crim_3UTR_M9-10-2_pA100 |
| 234, 235 | 267 | pT7_TL_5Pf_E2Crim_3UTR_M10-9-2_pA100 |
| 236, 237 | 268 | pT7_TL_5Pf_E2Crim_3UTR_M11-8-2_pA100 |
| 238, 239 | 269 | pT7_TL_5Pf_E2Crim_3UTR_M12-7-2_pA100 |
| 240, 241 | 270 | pT7_TL_5Pf_E2Crim_3UTR_M13-6-2_pA100 |
| 242, 243 | 271 | pT7_TL_5Pf_E2Crim_3UTR_M14-5-2_pA100 |
| 244, 245 | 272 | pT7_TL_5Pf_E2Crim_3UTR_M15-12-3_pA100 |
| 246, 247 | 273 | pT7_TL_5Pf_E2Crim_3UTR_M16-11-3_pA100 |
| 248, 249 | 274 | pT7_TL_6Pf_E2Crim_3UTR_M17-10-3_pA100 |
| 250, 251 | 275 | pT7_TL_5Pf_E2Crim_3UTR_M18-9-3_pA100 |
| 252, 253 | 276 | pT7_TL_5Pf_E2Crim_3UTR_M19-8-3_pA100 |
| 254, 255 | 277 | pT7_TL_5Pf_E2Crim_3UTR_M20-14-3_pA100 |
| 256, 257 | 278 | pT7_TL_5Pf_E2Crim_3UTR_M21-13-3_pA100 |
| 216, 217 | 279 | pT7_TL_5Pf_Spk_E2Crim_3UTR_M1-9-1_pA100 |
| 218, 219 | 280 | pT7_TL_5Pf_Spk_E2Crim_3UTR_M2-8-1_pA100 |
| 220, 221 | 281 | pT7_TL_5Pf_Spk_E2Crim_3UTR_M3-7-1_pA100 |
| 222, 223 | 282 | pT7_TL_5Pf_Spk_E2Crim_3UTR_M4-6-1_pA100 |
| 224, 225 | 283 | pT7_TL_5Pf_Spk_E2Crim_3UTR_M5-5-1_pA100 |
| 226, 227 | 284 | pT7_TL_5Pf_Spk_E2Crim_3UTR_M6-4-1_pA100 |

(continued)

| | | |
|---|---|---|
| 228, 229 | 285 | pT7_TL_5Pf_Spk_E2Crim_3UTR_M7-3-1_pA100 |
| 230, 231 | 286 | pT7_TL_5Pf_Spk_E2Crim_3UTR_M8-11-2_pA100 |
| 232, 233 | 287 | pT7_TL_5Pf_Spk_E2Crim_3UTR_M9-10-2_pA100 |
| 234, 235 | 288 | pT7_TL_5Pf_Spk_E2Crim_3UTR_M10-9-2_pA100 |
| 236, 237 | 289 | pT7_TL_5Pf_Spk_E2Crim_3UTR_M11-8-2_pA100 |
| 238, 239 | 290 | pT7_TL_5Pf_Spk_E2Crim_3UTR_M12-7-2_pA100 |
| 240, 241 | 291 | pT7_TL_5Pf_Spk_E2Crim_3UTR_M13-6-2_pA100 |
| 242, 243 | 292 | pT7_TL_5Pf_Spk_E2Crim_3UTR_M14-5-2_pA100 |
| 244, 245 | 293 | pT7_TL_5Pf_Spk_E2Crim_3UTR_M15-12-3_pA100 |
| 246, 247 | 294 | pT7_TL_5Pf_Spk_E2Crim_3UTR_M16-11-3_pA100 |

[Table 2-4]

Table 2-4

| | | |
|---|---|---|
| 248, 249 | 295 | pT7_TL_5Pf_Spk_E2Crim_3UTR_M17-10-3_pA100 |
| 250, 251 | 296 | pT7_TL_5Pf_Spk_E2Crim_3UTR_M18-9-3_pA100 |
| 252, 253 | 297 | pT7_TL_5Pf_Spk_E2Crim_3UTR_M19-8-3_pA100 |
| 254, 255 | 298 | pT7_TL_5Pf_Spk_E2Crim_3UTR_M20-14-3_pA100 |
| 256, 257 | 299 | pT7_TL_5Pf_Spk_E2Crim_3UTR_M21-13-3_pA100 |
| 252, 253 | 276 | pT7_TL_5Pf_E2Crim_3UTR_M19-8-3_pA100 |
| 254, 255 | 277 | pT7_TL_5Pf_E2Crim_3UTR_M20-14-3_pA100 |
| 256, 257 | 278 | pT7_TL_5Pf_E2Crim_3UTR_M21-13-3_pA100 |
| 216, 217 | 279 | pT7_TL_5Pf_Spk_E2Crim_3UTR_M1-9-1_pA100 |
| 218, 219 | 280 | pT7_TL_5Pf_Spk_E2Crim_3UTR_M2-8-1_pA100 |
| 220, 221 | 281 | pT7_TL_5Pf_Spk_E2Crim_3UTR_M3-7-1_pA100 |
| 222, 223 | 282 | pT7_TL_5Pf_Spk_E2Crim_3UTR_M4-6-1_pA100 |
| 224, 225 | 283 | pT7_TL_5Pf_Spk_E2Crim_3UTR_M5-5-1_pA100 |
| 226, 227 | 284 | pT7_TL_5Pf_Spk_E2Crim_3UTR_M6-4-1_pA100 |
| 228, 229 | 285 | pT7_TL_5Pf_Spk_E2Crim_3UTR_M7-3-1_pA100 |
| 230, 231 | 286 | pT7_TL_5Pf_Spk_E2Crim_3UTR_M8-11-2_pA100 |
| 232, 233 | 287 | pT7_TL_5Pf_Spk_E2Crim_3UTR_M9-10-2_pA100 |
| 234, 235 | 288 | pT7_TL_5Pf_Spk_E2Crim_3UTR_M10-9-2_pA100 |
| 236, 237 | 289 | pT7_TL_5Pf_Spk_E2Crim_3UTR_M11-8-2_pA100 |
| 238, 239 | 290 | pT7_TL_5Pf_Spk_E2Crim_3UTR_M12-7-2_pA100 |
| 240, 241 | 291 | pT7_TL_5Pf_Spk_E2Crim_3UTR_M13-6-2_pA100 |
| 242, 243 | 292 | pT7_TL_5Pf_Spk_E2Crim_3UTR_M14-5-2_pA100 |
| 244, 245 | 293 | pT7_TL_5Pf_Spk_E2Crim_3UTR_M15-12-3_pA100 |
| 246, 247 | 294 | pT7_TL_5Pf_Spk_E2Crim_3UTR_M16-11-3_pA100 |
| 248, 249 | 295 | pT7_TL_5Pf_Spk_E2Crim_3UTR_M17-10-3_pA100 |
| 250, 251 | 296 | pT7_TL_5Pf_Spk_E2Crim_3UTR_M18-9-3_pA100 |

(continued)

| 252, 253 | 297 | pT7_TL_5Pf_Spk_E2Crim_3UTR_M19-8-3_pA100 |
| 254, 255 | 298 | pT7_TL_5Pf_Spk_E2Crim_3UTR_M20-14-3_pA100 |
| 256, 257 | 299 | pT7_TL_5Pf_Spk_E2Crim_3UTR_M21-13-3_pA100 |

The 5' UTR sequence of the HSD17B4 gene was obtained by annealing synthetic oligonucleotides set forth in SEQ ID NOs: 99 and 100. The DNA fragment was inserted into a BsaI site of pT7_TL_E2Crimson_pA100 to construct pT7_TL_5HSD_E2Crim_pA100 plasmid. A 3' UTR sequence partially complementary to the 5' UTR sequence of the HSD17B4 gene was obtained by annealing synthetic oligonucleotides described below. The DNA fragment was inserted into a PaqCI site of pT7_TL_5HSD_E2Crim_pA100 to construct pT7_TL_5HSD_E2Crim_3UTR_pA100 plasmid. The combinations of the sequence numbers of the synthetic oligonucleotides and the constructed plasmids are as follows.

[Table 3]

**Table 3**

| Synthetic oligonucleotide SEQ ID NO | Plasmid SEQ ID NO | Plasmid name |
|---|---|---|
| 101, 102 | 178 | pT7_TL_5HSD_E2Crim_3UTR-9-1_pA100 |
| 103, 104 | 179 | pT7_TL_5HSD_E2Crim_3UTR-8-1_pA100 |
| 105, 106 | 180 | pT7_TL_5HSD_E2Crim_3UTR-7-1_pA100 |
| 107, 108 | 181 | pT7_TL_5HSD_E2Crim_3UTR-6-1_pA100 |
| 109, 110 | 182 | pT7_TL_5HSD_E2Crim_3UTR-5-1_pA100 |
| 111, 112 | 183 | pT7_TL_5HSD_E2Crim_3UTR-4-1_pA100 |
| 113, 114 | 184 | pT7_TL_5HSD_E2Crim_3UTR-3-1_pA100 |
| 115, 116 | 185 | pT7_TL_5HSD_E2Crim_3UTR-9-2_pA100 |
| 117, 118 | 186 | pT7_TL_5HSD_E2Crim_3UTR-8-2_pA100 |
| 119, 120 | 187 | pT7_TL_5HSD_E2Crim_3UTR-7-2_pA100 |
| 121, 122 | 188 | pT7_TL_5HSD_E2Crim_3UTR-6-2_pA100 |
| 123, 124 | 189 | pT7_TL_5HSD_E2Crim_3UTR-5-2_pA100 |
| 125, 126 | 190 | pT7_TL_5HSD_E2Crim_3UTR-12-3_pA100 |
| 127, 128 | 191 | pT7_TL_5HSD_E2Crim_3UTR-11-3_pA100 |
| 129, 130 | 192 | pT7_TL_5HSD_E2Crim_3UTR-10-3_pA100 |
| 131, 132 | 193 | pT7_TL_5HSD_E2Crim_3UTR-9-3_pA100 |
| 133, 134 | 194 | pT7_TL_5HSD_E2Crim_3UTR-8-3_pA100 |

(In vitro transcription by T7RNA polymerase)

In order to stop RNA synthesis by dissociating the RNA polymerase from the template DNA and to make the 3' end of the synthesized RNA a continuous A nucleotide sequence, the plasmid serving as the template DNA was linearized by a restriction enzyme BsmBI (New England Biolab). The plasmid after the BsmBI treatment was sufficiently mixed by adding TE saturated phenol, and then separated into an organic phase and an aqueous phase by centrifugation (20,000 × g, 10 minutes), and the aqueous phase was recovered in a new tube. The recovered aqueous phase was separated into a 1-butanol phase and an aqueous phase by centrifugation (20,000 × g, 10 minutes) after addition of 1-butanol and sufficient mixing, and the separated 1-butanol phase was removed. By repeating the operation three times, phenol was completely removed and the volume of the aqueous phase was reduced. To the aqueous phase, a 3 M sodium acetate solution (pH 5.2) (NACALAI TESQUE, INC.) and ethanol were added and sufficiently mixed, and then centrifuged (20,000 × g, 10 minutes) to precipitate DNA fragments. The precipitated DNA fragment was washed with 70% ethanol and then dissolved in distilled water to obtain a linear template DNA. An in vitro transcription reaction was performed by incubating at 42°C for 3 hours under the conditions of 25 ng/$\mu$l linear template DNA, 1 × T7RNA polymerase attached buffer (Takara Bio Inc.), 5 mM DTT, 1.6 mM CleanCap (TriLink), 0.4 mM GTP, 2.0 mM ATP, 2.0 mM CTP and 2.0 mM N1-methyl-pseudo-UTP (TriLink or Yamasa Corporation), 1 U/$\mu$l Recombinant RNase Inhibitor (Takara Bio Inc.), 2 U/ml inorganic pyrophosphatase (New England Biolab), and 2.5 U/$\mu$l T7RNA polymerase (Takara Bio Inc.). After the reaction, in order to remove the remaining linear template DNA, Recombinant DNaseI (Takara Bio Inc.) was added so as to have a final concentration of 0.125 U/$\mu$l, and further incubated at 37°C for 15 minutes. The solution containing RNA after the reaction was sufficiently mixed by adding a 1:1 mixed solution of citric acid saturated phenol (pH 4.3) (NACALAI TESQUE, INC.) and chloroform (NACALAI TESQUE, INC.), and then separated into an organic phase and an aqueous phase by centrifugation (20,000 × g, 10 minutes). Chloroform was added to the recovered aqueous phase and sufficiently mixed, and then the mixture was separated into a chloroform phase and an aqueous phase by centrifugation (20,000 × g, 10 minutes). The operation was repeated twice to completely remove phenol contained in the aqueous phase. To the aqueous phase, a 3 M sodium acetate solution (pH 5.2) and ethanol were added and sufficiently mixed, and then centrifuged (20,000 × g, 10 minutes) to precipitate RNA. The precipitated RNA was washed with 70% ethanol and then dissolved in 50 $\mu$l of distilled water. The RNA solution was applied to MicroSpin S-200 Column (Cytiva) previously centrifuged at 700 × g for 1 minute, centrifuged at 700 × g for 2 minutes, and further centrifuged at 700 × g for 2 minutes after addition of 50 $\mu$l of distilled water to remove unreacted nucleotides contained in the RNA solution. To the eluate, a 3 M sodium acetate solution (pH 5.2) and ethanol were added and sufficiently mixed, and then centrifuged (20,000 × g, 10 minutes) to precipitate RNA. The precipitated RNA was washed with 70% ethanol and then dissolved in 20 $\mu$l of distilled water. The RNA concentration was quantified with a microvolume spectrophotometer Nano drop One (Thermofisher) and Qubit RNA Broad Range Assay Kit (Thermofisher) according to the manufacturer's instruction manual.

(Quantitative example)

(RNA transfection into cultured cells and quantification of fluorescent protein expression level)

293 cells were cultured in DMEM (Thermofisher or NACALAI TESQUE, INC.) to which 10% FBS (Thermofisher) and a penicillin-streptomycin mixed solution (NACALAI TESQUE, INC.) were added at 37°C in the presence of 5% $CO_2$. 1 × $10^5$ cells were seeded per well in a 6-well culture plate with a 1.5 ml of a culture medium, cultured at 37°C for 24 hours, and then subjected to RNA transfection. A mixture of 48.5 $\mu$l of Opti-MEM (Thermofisher) and 1.5 $\mu$l of Lipofectamine messengerMAX (Thermofisher) and 50 $\mu$l of Opti-MEM containing 50 ng of RNA were each prepared in advance per well, mixed, and incubated at room temperature for 10 minutes. The solution was added to the culture well and further cultured at 37°C for 24 hours. The 293 cells after RNA transfection were washed with 1 ml of DPBS (Thermofisher) per well and then dissociated from the bottom of the culture plate with 0.5 ml of trypsin-EDTA (NACALAI TESQUE, INC.). The dissociated cells were added with 1 ml of DPBS and transferred to a 1.5 ml tube, and then recovered by centrifugation at 3,000 × g for 3 minutes. The recovered cells were resuspended in 100 $\mu$l of DPBS, and 30$\mu$L of the suspension was dispensed into a black flat-bottom 384-well microplate (Greiner). The relative amount of E2Crimson protein contained in each well was calculated by fluorescence measurement at an excitation wavelength of 606 nm and a fluorescence wavelength of 651 nm by a plate reader Infinite 200 PRO (TECAN). Furthermore, after the fluorescence measurement, 3 $\mu$l of DPBS containing 5 $\mu$g/ml of Calcein-AM (DOJINDO LABORATORIES) was added to and mixed with the cell suspension dispensed to each well, and the mixture was incubated at 37°C for 30 minutes. The relative viable cell number contained in each well was calculated by fluorescence measurement at an excitation wavelength of 480 nm and a fluorescence wavelength of 533 nm indicated by Calcein, which is a hydrolysis product of Calcein-AM by intracellular esterase. Note that it has been further confirmed that fluorescence indicated by the E2Crimson protein does not interfere with fluorescence indicated by Calcein. A ratio of an E2Crimson fluorescence measurement value to a Calcein fluorescence measurement value was defined as a relative expression level of E2Crimson protein in each mRNA.

(Example 1: expression efficiency by complementarity between 5'-3' untranslated regions: utility of non-complementary 2 and 3 bases)

In the present example, the expression level of mRNA in which the 5' UTR was a GAPDH gene sequence, a Pfizer sequence, or an HSD17B4 gene sequence was examined. For comparison, mRNAs in which both the 5' UTR and 3' UTR were the Pfizer sequences were used. The production of the construct and the quantification of the expression level were performed on the basis of the production examples and quantitative examples described above.

(Results)

The results are illustrated in FIG. 1. A ratio of an E2Crimson fluorescence measurement value to a Calcein fluorescence measurement value was defined as a relative expression level of E2Crimson protein in each mRNA. A plurality of samples were measured for each mRNA; the average value is shown in a bar graph, and the individual values of each sample are shown as black dots. In the case of (the number of complementary bases)/(the number of non-complementary bases) = 3, when the 5' UTR was GAPDH or HSD17B4, a higher expression level was shown at two or three non-complementary bases. When the 5' UTR was Pfizer, the results were almost equivalent at non-complementary 1 to 3 bases.

FIG. 2 illustrates the results of examining an expression level of mRNA in which a 5' UTR is a GAPDH gene sequence, and a 3' UTR is a sequence having a degree of complementarity of 100%, 94%, or 75% to the 5' UTR. A ratio of an E2Crimson fluorescence measurement value to a Calcein fluorescence measurement value was defined as a relative expression level of E2Crimson protein in each mRNA. A plurality of samples were measured for each mRNA; the average value is shown in a bar graph, and the individual values of each sample are shown as black dots. The highest expression level was observed when the non-complementary region was 2 bases (#2), and the expression levels were similar when the non-complementary region was 1 base and 4 bases. When the complementarity exceeded 90%, the expression level decreased. In addition, when the non-complementary region exceeded 4 bases, the expression level decreased.

(Example 2: expression efficiency by complementarity between 5'-3' untranslated regions: 5' UTR GAPDH)

In the present example, an expression level was examined in 293 cells for mRNA having a GAPDH gene sequence as a 5' UTR and various sequences having partial complementarity to the 5' UTR as a 3' UTR. The production and quantification of the construct were performed on the basis of the production examples and quantitative examples described above.

(Results)

FIG. 3 illustrates base sequences of a 3' UTR and a 5' UTR used in the present example. The results are illustrated in FIG. 4. For comparison, mRNAs in which both the 5' UTR and 3' UTR were the Pfizer sequences were used. A ratio of an E2Crimson fluorescence measurement value to a Calcein fluorescence measurement value was defined as a relative expression level of E2Crimson protein in each mRNA. A plurality of samples were measured for each mRNA; the average value is shown in a bar graph, and the individual values of each sample are shown as black dots. When the non-complementary region was 1 base, a high expression level was shown at a degree of complementarity of 75 to 89% and a complementary region of 3 to 7 bases. When the complementary region exceeded 7 bases, the expression level decreased. In addition, when the non-complementary region was 2 bases and the complementary region was 6 bases, and when the non-complementary region was 3 bases and the complementary region was 11 bases, the expression level was high.

(Example 3: expression efficiency by complementarity between 5'-3' untranslated regions: 5' UTR Pfizer sequence)

In the present example, an expression level was examined in 293 cells for mRNA having a Pfizer sequence as a 5' UTR and various sequences having partial complementarity to the 5' UTR as a 3' UTR. The production and quantification of the construct were performed on the basis of the production examples and quantitative examples described above.

(Results) FIG. 5 illustrates base sequences of a 3' UTR and a 5' UTR used in the present example. The results are illustrated in FIG. 6. For comparison, mRNAs in which both the 5' UTR and 3' UTR were the Pfizer sequences were used. A ratio of an E2Crimson fluorescence measurement value to a Calcein fluorescence measurement value was defined as a relative expression level of E2Crimson protein in each mRNA. A plurality of samples were measured for each mRNA; the average value is shown in a bar graph, and the individual values of each sample are shown as black dots. In mRNA with the complementarity/non-complementarity of 4-1, 3-1, 6-2, 5-2, or 9-3, the expression level was higher than that of the Pfizer sequence with an increase of up to approximately 50%. When the number of complementary bases was short, the expression level tended to be high.

(Example 4: expression efficiency by complementarity between 5'-3' untranslated regions: 5' UTR HSD17B4 sequence)

In the present example, an expression level was examined in 293 cells for mRNA having an HSD17B4 gene sequence as a 5' UTR and various sequences having partial complementarity to the 5' UTR as a 3' UTR. The production and quantification of the construct were performed on the basis of the production examples and quantitative examples described above.

(Results)

FIG. 7 illustrates base sequences of a 3' UTR and a 5' UTR used in the present example, and the results are illustrated in FIG. 8. For comparison, mRNAs in which both the 5' UTR and 3' UTR were the Pfizer sequences were used. A ratio of an E2Crimson fluorescence measurement value to a Calcein fluorescence measurement value was defined as a relative expression level of E2Crimson protein in each mRNA. A plurality of samples were measured for each mRNA; the average value is shown in a bar graph, and the individual values of each sample are shown as black dots. In mRNA with the complementarity/non-complementarity of 4-1, 3-1, 6-2, 5-2, 9-3, or 8-3, the expression level was higher than that of the Pfizer sequence.

To summarize the above, when the non-complementary region is 1 base, the complementary region is preferably 3 to 7 bases, when the non-complementary region is 2 bases, the complementary region is preferably 5 to 7 bases, and when the non-complementary region is 3 bases, the complementary region is preferably 8 to 11 bases.

(Example 5: expression efficiency by degree of complementarity between 5'-3' untranslated regions: critical significance at complementarity of 75 to 80%)

In the present example, an expression level was examined in 293 cells for mRNA having a GAPDH gene sequence as a 5' UTR and various sequences having partial complementarity to the 5' UTR as a 3' UTR. The production and quantification of the construct were performed on the basis of the production examples and quantitative examples described above. The combinations of the sequence numbers of the synthetic oligonucleotides and the constructed plasmids are as follows.

[Table 4]

**Table 4**

| Synthetic oligonucleotide SEQ ID NO | Plasmid SEQ ID NO | Plasmid name |
|---|---|---|
| 195, 196 | 209 | pT7_TL_5GAP_E2Crim_3UTR_24-4/3-1_pA100 |
| 197, 198 | 210 | pT7_TL_5GAP_E2Crim_3UTR_25-4/3-1_pA100 |
| 199, 200 | 211 | pT7_TL_5GAP_E2Crim_3UTR_26-4/3-1_pA100 |
| 201, 202 | 212 | pT7_TL_5GAP_E2Crim_3UTR_27-4/3-1_pA100 |
| 203, 204 | 213 | pT7_TL_5GAP_E2Crim_3UTR_28-4/3-1_pA100 |
| 205, 206 | 214 | pT7_TL_5GAP_E2Crim_3UTR_29-4/3-1_pA100 |
| 207, 208 | 215 | pT7_TL_5GAP_E2Crim_3UTR_30-4/3-1_pA100 |

(Results)

FIG. 9 illustrates base sequences of a 3' UTR and a 5' UTR used in the present example, and the results are illustrated in FIG. 10. For comparison, mRNAs in which both the 5' UTR and 3' UTR were the Pfizer sequences were used. A ratio of an E2Crimson fluorescence measurement value to a Calcein fluorescence measurement value was defined as a relative expression level of E2Crimson protein in each mRNA. Three samples were measured for each mRNA, and the average value is shown as a plot. FIG. 10 illustrates that the degree of complementarity is maximized between 75% and 80%, indicating that a degree of complementarity of 75% or higher is preferable.

(Example 6: expression efficiency by complementarity between 5'-3' untranslated regions: change in free energy)

In the present example, a correlation between a change in free energy in interaction between a 5' UTR and a 3' UTR and an expression level of each mRNA was examined for mRNA in which a 3' UTR has partial complementarity to a 5' UTR. The production and quantification of the construct were performed on the basis of the production examples and quantitative examples described above.

(Results)

The results are illustrated in FIG. 11. The expression level was calculated with mRNA of a Pfizer sequence as 1 for both the 5' UTR and the 3' UTR. The change in free energy was obtained by the secondary structure prediction program Mfold (Michael Zuker, Mfold web server for nucleic acid folding and hybridization prediction, Nucleic Acids Res. 2003 Jul 1;31(13):3406-15). The left side, center, and right side of the drawing show the results when the 5' UTRs are those of a GAPDH gene, an HSD17B4 gene, and a Pfizer sequence, respectively. For each 5' UTR, the obtained measured value was fitted to a 4-parameter logistic curve $y = d + (a-d)/(1+ (x/c)^b)$, which is one of the sigmoid curves. FIG. 11 reveals that there is a correlation between the change in free energy and the expression level, and that the expression level can be predicted to some extent from the change in free energy.

FIG. 12 is illustrated. The maximum value of the change in free energy was defined as a value of the change in free energy calculated by Mfold for an mRNA in which base substitutions were introduced in the entire 3' UTR to create mismatches with the 5' UTR, where the degree of complementarity between the 5' UTR and 3' UTR was 100%. The relative value of the change in free energy was calculated with the rate of increase (right shift) from the inflection point as an index and, where the difference between the value at the inflection point and the maximum value as 100%. The expression level of mRNA was the highest when the value of the change in free energy represented an increase of 10% or more and 50% or less from the inflection point.

FIG. 13 illustrates a method for predicting an expression level of mRNA from 5' UTR and 3' UTR sequences. The maximum value and the minimum value of the change in free energy were obtained in the same manner as in FIG. 12. It was found that the average value of the maximum value and the minimum value of the free energy was consistent with the value of the change in free energy that becomes the inflection point of the sigmoid curve. By using the average value, the inflection point could be predicted with an accuracy of an error of 4.9 kcal/mol or less or 13.1% or less. Similarly to FIG. 12, the error percentage is calculated by taking the difference between the value to be the inflection point and the maximum value of the change in free energy as 100%. Based on the prediction of the inflection point, the change in free energy of the interaction between the 5' UTR and the 3' UTR in mRNA at which the expression level is maximized can be predicted.

(Example 7: prediction of expression level using change in free energy)

1. A candidate sequence of a 5' UTR is designed.

2. A 3' UTR candidate sequence that is partially complementary (degree of complementarity: 40 to 90%) to the 5' UTR is designed to be non-complementary 1, 2, or 3 bases and to be complementary 4 to 14 bases. At this time, the number of complementary bases is a continuous number. For example, when complementary 7 to 10 bases are selected, all sequences having complementary 7, 8, 9, and 10 bases are designed. For each type of 5' UTR, 10 to 20 kinds of 5' UTRs are designed.

3. The change in free energy of the designed sequence is obtained by the secondary structure prediction program Mfold.

4. The average of the maximum value and the minimum value of the change in free energy is used as a reference value. Here, the maximum value of the change in free energy is based on a sequence in which mismatch substitution is performed for all 3' UTR bases, and the minimum value of the change in free energy is based on a sequence having 100% 5'-3' UTR complementarity.

5. Candidate sequences having a value of a change in free energy in a range of a rate of increase of 0% to 60% with respect to the reference value are selected.

6. If necessary, the protein expression level of the selected candidate sequence is confirmed, and a sequence having a high expression level is selected.

(Example 8: expression efficiency by complementarity between 5'-3' untranslated regions: myoblast cell line C2C12)

In the present example, an expression level was examined in C2C12 cells for mRNA having a Pfizer sequence as a 5' UTR and various sequences having partial complementarity to the 5' UTR as a 3' UTR. The production and quantification of the construct were performed on the basis of the production examples and quantitative examples described above.

(Results)

FIG. 5 illustrates base sequences of a 3' UTR and a 5' UTR used in the present example. The results are illustrated in FIG. 15. For comparison, mRNAs in which both the 5' UTR and 3' UTR were the Pfizer sequences were used.

A ratio of an E2Crimson fluorescence measurement value to a Calcein fluorescence measurement value was defined as a relative expression level of E2Crimson protein in each mRNA. A plurality of samples were measured for each mRNA; the average value is shown in a bar graph, and the individual values of each sample are shown as black dots. In mRNA with the complementarity/non-complementarity of 4-1, 3-1, 6-2, 5-2, or 9-3, the expression level was higher than that of the Pfizer sequence with an increase of up to approximately 60% Since the results were comparable to those in 293 cells as illustrated in FIG. 6, it was revealed that the effects of the partially complementary 5' untranslated region (UTR) and 3' UTR are not limited to cell types.

(Example 9: expression efficiency by complementarity between 5'-3' untranslated regions: long chain mRNA)

In the present example, an expression level in 293 cells was examined for mRNA having a Pfizer sequence as a 5' UTR, various sequences having partial complementarity with the 5' UTR as a 3' UTR, and SARS CoV2 Spike-E2Crimson fusion protein as ORF. The production and quantification of the construct were performed on the basis of the production examples and quantitative examples described above.

(Results)

FIG. 5 illustrates base sequences of a 3' UTR and a 5' UTR used in the present example. The results are illustrated in FIG. 16. For comparison, mRNAs in which both the 5' UTR and 3' UTR were the Pfizer sequences were used. A ratio of an E2Crimson fluorescence measurement value to a Calcein fluorescence measurement value was defined as a relative expression level of E2Crimson protein in each mRNA. A plurality of samples were measured for each mRNA; the average value is shown in a bar graph, and the individual values of each sample are shown as black dots. In the complementarity/non-complementarity of 6-1, 3-1, 11-2, 8-2, 7-2, 6-2, 5-2, 12-3, 11-3, 10-3, 9-3, or 8-3, the expression level was higher than that of the Pfizer sequence with an increase of up to approximately 2-fold. The result was equivalent to the result in mRNA using the E2Crimson protein as ORF as illustrated in FIG. 6. Since the ORF of the SARS CoV2 Spike-E2Crimson fusion protein is 4.5 kbp and the ORF of the E2Crimson protein is 0.7 kbp, it was revealed that the effect of the partially complementary 5' untranslated region (UTR) and 3' UTR is not limited to the type and length of ORF.

(Example 10: expression efficiency by complementarity between 5'-3' untranslated regions: base removal in non-complementary region)

In the present example, an expression level was examined in 293 cells for mRNA having a Pfizer sequence as a 5' UTR and various sequences having partial complementarity to the 5' UTR as a 3' UTR. In these 3' UTRs, the base is removed to form a non-complementary sequence. The production and quantification of the construct were performed on the basis of the production examples and quantitative examples described above.

(Results)

FIG. 17(2) illustrates mRNA used in the present example as a schematic diagram. FIG. 18 illustrates base sequences of a 3' UTR and a 5' UTR used in the present example. The results of mRNA from which a non-complementary region on the 3' UTR was removed as illustrated in FIG. 18(2) are illustrated in FIGS. 19 and 20. For comparison, mRNAs in which both the 5' UTR and 3' UTR were the Pfizer sequences were used. A ratio of an E2Crimson fluorescence measurement value to a Calcein fluorescence measurement value was defined as a relative expression level of E2Crimson protein in each mRNA. A plurality of samples were measured for each mRNA; the average value is shown in a bar graph, and the individual values of each sample are shown as black dots. In the complementarity/non-complementarity of 9-1, 5-1, 4-1, 3-1, 9-2, 8-2, 7-2, 6-2, 5-2, 11-3, 10-3, 9-3, or 8-3, the expression level was higher than that of the Pfizer sequence with an increase of up to approximately 60%. When the non-complementary region illustrated in FIG. 6 was used as a base substitution, the expression level was higher than that of the Pfizer sequence in the mRNA with complementarity/non-complementarity of 4-1, 3-1, 6-2, 5-2, 9-3, or 8-3. Therefore, in the case of base removal, a tendency for higher expression levels was observed in mRNAs having longer complementary strands and a high degree of complementarity. Furthermore, the expression levels of mRNA from which a non-complementary region on the 5' UTR is removed as illustrated in FIG. 18(3), mRNA in which base substitution and base removal are mixed as illustrated in FIG. 18(4), mRNA in which a non-complementary sequence is created by adding a base in the 3' UTR as illustrated in FIG. 18(5), and mRNA in which a non-complementary sequence is created by adding a base in the 5' UTR as illustrated in FIG. 18(6) are also confirmed by the same method. The sequences of plasmids used for mRNA synthesis are shown in SEQ ID NOs: 301 to 315 and 334 to 339. The expression level can be regulated similarly to the mRNA in which the non-complementary region on the 3' UTR is removed as illustrated in FIG. 18(2).

[Table 5-1]

**Table 5**

| SEQ ID NO | Name |
|---|---|
| 301 | pT7_TL_5Pf_E2Crim_3UTR-8-2-1_pA100 |
| 302 | pT7_TL_5Pf_E2Crim_3UTR-8-2-2_pA100 |
| 303 | pT7_TL_5Pf_E2Crim_3UTR-8-2-3_pA100 |
| 304 | pT7_TL_5Pf_E2Crim_3UTR-8-2-4__pA100 |
| 305 | pT7_TL_5Pf_E2Crim_3UTR-8-2-5_pA100 |
| 306 | pT7_TL_5Pf_E2Crim_3UTR-8-2-6_pA100 |
| 307 | pT7_TL_5Pf_E2Crim_3UTR-8-2-7_pA100 |
| 308 | pT7_TL_5Pf_E2Crim_3UTR-8-2-8_pA100 |
| 309 | pT7_TL_5Pf_E2Crim_3UTR-8-2-9_pA100 |
| 310 | pT7_TL_SPf_E2Crim_3UTR-8-2-10_pA100 |
| 311 | pT7_TL_5Pf_E2Crim_3UTR-8-2-11_pA100 |
| 312 | pT7_TL_5Pf_E2Crim_3UTR-8-2-12_pA100 |
| 313 | pT7_TL_5Pf_E2Crim_3UTR-8-2-13_pA100 |
| 314 | pT7_TL_5Pf_E2Crim_3UTR-8-2-14_pA100 |
| 315 | pT7_TL_5Pf_E2Crim_3UTR-8-2-15_pA100 |
| 316 | pT7_TL_5Pf_E2Crim_3UTR-3-1/6-2-1_pA100 |
| 317 | pT7_TL_5Pf_E2Crim_3UTR-3-1/6-2-2_pA100 |
| 318 | pT7_TL_5Pf_E2Crim_3UTR-6-2/9-3-1_pA100 |
| 319 | pT7_TL_5Pf_E2Crim_3UTR-6-2/9-3-2_pA100 |

[Table 5-2]

| | |
|---|---|
| 320 | pT7_TL_5Pf_E2Crim_3UTR-3-1/9-3-1_pA100 |
| 321 | pT7_TL_5Pf_E2Crim_3UTR-3-1/9-3-2_pA100 |
| 322 | pT7_TL_5Pf_E2Crim_3UTR-9-3/9-2-1_pA100 |
| 323 | pT7_TL_5Pf_E2Crim_3UTR-9-3/9-2-2_pA100 |
| 324 | pT7_TL_5Pf_E2Crim_3UTR-9-3/9-1-1_pA100 |
| 325 | pT7_TL_5Pf_E2Crim_3UTR-9-3/9-1-2_pA100 |
| 326 | pT7_TL_5Pf_E2Crim_3UTR-8-2/8-1-1_pA100 |
| 327 | pT7_TL_5Pf_E2Crim_3UTR-8-2/8-1-2_pA100 |
| 328 | pT7_TL_5Pf_E2Crim_3UTR-9-3/9-2-3_pA100 |
| 329 | pT7_TL_5Pf_E2Crim_3UTR-9-3/9-2-4_pA100 |
| 330 | pT7_TL_5Pf_E2Crim_3UTR-9-3/9-1-3_pA100 |
| 331 | pT7_TL_5Pf_E2Crim_3UTR-9-3/9-1-4_pA100 |
| 332 | pT7_TL_5Pf_E2Crim_3UTR-8-2/8-1-3_pA100 |
| 333 | pT7_TL_5Pf_E2Crim_3UTR-8-2/8-1-4_pA100 |
| 334 | pT7_TL_5Pf_E2Crim_3UTR-8-1-A1_pA100 |
| 335 | pT7_TL_5Pf_E2Crim_3UTR-8-2-A2_pA100 |

(continued)

| 336 | pT7_TL_5Pf_E2Crim_3UTR-8-3-A3_pA100 |
| 337 | pT7_TL_5Pf_E2Crim_3UTR-8-1-B1_pA100 |
| 338 | pT7_TL_5Pf_E2Crim_3UTR-8-2-B2_pA100 |
| 339 | pT7_TL_5Pf_E2Crim_3UTR-8-3-B3_pA100 |

(Example 11: expression efficiency by complementarity between 5'-3' untranslated regions: base removal in non-complementary region and myoblast cell line C2C12)

In the present example, an expression level was examined in C2C12 cells for mRNA having a Pfizer sequence as a 5' UTR and various sequences having partial complementarity to the 5' UTR as a 3' UTR. In these 3' UTRs, the base is removed to form a non-complementary sequence. The production and quantification of the construct were performed on the basis of the production examples and quantitative examples described above.

(Results)

The results obtained from the mRNAs used in FIGS. 19 and 20 are illustrated in FIGS. 21 and 22. For comparison, mRNAs in which both the 5' UTR and 3' UTR were the Pfizer sequences were used. A ratio of an E2Crimson fluorescence measurement value to a Calcein fluorescence measurement value was defined as a relative expression level of E2Crimson protein in each mRNA. A plurality of samples were measured for each mRNA; the average value is shown in a bar graph, and the individual values of each sample are shown as black dots. In the complementarity/non-complementarity of 4-1, 3-1, 8-2, 7-2, 6-2, 5-2, 11-3, 10-3, 9-3, or 8-3, the expression level was higher than that of the Pfizer sequence with an increase of up to approximately 50%. When the non-complementary region illustrated in FIG. 15 was used as a base substitution, the expression level was higher than that of the Pfizer sequence in the mRNA with complementarity/non-complementarity of 4-1, 3-1, 6-2, 5-2, or 9-3. Therefore, as in the case of 293 cells, in the case of base removal, a tendency for higher expression levels was observed in mRNAs having longer complementary strands and a high degree of complementarity.

(Example 12: expression efficiency by complementarity between 5'-3' untranslated regions: base removal in non-complementary region, myoblast cell line C2C12, and long chain mRNA)

In the present example, an expression level was examined in C2C12 cells for mRNA having a Pfizer sequence as a 5' UTR and various sequences having partial complementarity to the 5' UTR as a 3' UTR. In these 3' UTRs, the base is removed to form a non-complementary sequence. In addition, ORF is SARS CoV2 Spike-E2Crimson fusion protein. The production and quantification of the construct were performed on the basis of the production examples and quantitative examples described above.

(Results)

The results are illustrated in FIG. 23. For comparison, mRNAs in which both the 5' UTR and 3' UTR were the Pfizer sequences were used. A ratio of an E2Crimson fluorescence measurement value to a Calcein fluorescence measurement value was defined as a relative expression level of E2Crimson protein in each mRNA. A plurality of samples were measured for each mRNA; the average value is shown in a bar graph, and the individual values of each sample are shown as black dots. In the complementarity/non-complementarity of 9-1, 6-1, 5-1, 11-2, 10-2, 9-2, 8-2, 7-2, 11-3, 10-3, or 8-3, the expression level was higher than that of the Pfizer sequence with an increase of up to approximately 40%. When the non-complementary region illustrated in FIG. 16 was used as a base substitution, the expression level was higher than that of the Pfizer sequence in the mRNAs with complementarity/non-complementarity of 6-1, 11-2, 8-2, 7-2, 6-2, 5-2, 12-3, 11-3, 10-3, 9-3, or 8-3. Therefore, as in the case of long chain mRNA, in the case of base removal, a tendency for higher expression levels was observed in mRNAs having longer complementary strands and a high degree of complementarity.

(Example 13: expression efficiency by complementarity between 5'-3' untranslated regions: case of mixed non-complementary regions having different numbers of bases)

FIG. 24 illustrates base sequences of a 3' UTR and a 5' UTR used in the present example. The sequences of plasmids used for mRNA synthesis are shown in SEQ ID NOs: 316 to 333. FIG. 24(1) illustrates a case in which the non-complementary regions are all substituted, that is, the number of bases of the non-complementary regions is the same in the 5' UTR and the 3' UTR. The expression of the protein is confirmed by the method described in the above examples. The expression level may be regulated in the same manner as the mRNA illustrated in FIG. 5. Further, FIG. 24(2) illustrates a case in which the base of the non-complementary region is subject to substitution or removal, that is, the number of bases of the non-complementary region differs between the 5' UTR and the 3' UTR. The expression of the protein is confirmed by the method described in the above examples. The expression level can be regulated similarly to the mRNA in which the non-complementary region on the 3' UTR is removed as illustrated in FIG. 18(2).

(Note)

As described above, although the present disclosure is exemplified by the use of preferred embodiments of the present disclosure, it is understood that the scope of the present disclosure should be interpreted solely based on the claims. It is also understood that any patent, any patent application, and any references cited herein should be incorporated herein by reference in the same manner as the contents are specifically described herein. The present application claims priority to Japanese Patent Application No. 2023-013290, filed on January 31, 2023 to the Japan Patent Office, the entire contents of which are incorporated herein by reference as necessary.

Industrial Applicability

The present disclosure may be applied in an application field (for example, a medicine) of nucleic acid technology.

Sequence Listing Free Text

SEQ ID NOs: 1 to 339: See Table 1 in the specification.

**Claims**

1. A nucleic acid construct comprising a 5' untranslated region (UTR) and a 3' UTR that are at least partially complementary to each other, wherein at least one of UTRs includes a non-complementary region and a complementary region with respect to the other UTR, and when all of the respective lengths of the non-complementary regions are one base substitutions, a degree of complementarity of the 3' UTR to the 5' UTR is greater than 75%.

2. The nucleic acid construct according to claim 1, wherein the base of the non-complementary region is subject to substitution or removal.

3. The nucleic acid construct according to any one of claims 1 and 2, wherein at least one of the non-complementary regions comprises a length of 2 or more bases.

4. The nucleic acid construct according to any one of claims 1 to 3, wherein the respective length(s) of at least one of the non-complementary regions is/are of 2 bases or 3 bases.

5. The nucleic acid construct according to any one of claims 1 to 4, wherein the length of at least one of the complementary regions is of 5 or more bases.

6. The nucleic acid construct according to any one of claims 1 to 5, wherein the length of at least one of the complementary regions is of 5 to 11 bases.

7. The nucleic acid construct according to any one of claims 1 to 6, wherein the length of the non-complementary region is of 2 bases, and the length(s) of the complementary region(s) is/are each independently of 5 to 7 bases.

8. The nucleic acid construct according to any one of claims 1 to 6, wherein the length of the non-complementary region is of 3 bases, and the length(s) of the complementary region(s) is/are each independently of 8 to 11 bases.

9. The nucleic acid construct according to any one of claims 1 to 8, wherein all of the respective lengths of the non-complementary regions are each of 1 or more bases, and the degree of complementarity is greater than 75%.

10. The nucleic acid construct according to claim 9, wherein the degree of complementarity is 80% to 90%.

11. The nucleic acid construct according to claim 9 or 10, wherein the degree of complementarity is 81% to 89%.

12. The nucleic acid construct according to any one of claims 1 to 11, wherein at least one of the non-complementary regions is 1 base.

13. The nucleic acid construct according to any one of claims 9 to 12, wherein all of the respective lengths of the complementary regions are each independently of 3 to 11 bases.

14. The nucleic acid construct according to claim 9, wherein the non-complementary region is 1 base, and lengths of the complementary regions are each independently of 3 to 7 bases.

15. The nucleic acid construct according to claim 9, wherein a length of the non-complementary region is of 2 bases, and lengths of the complementary regions are each independently of 5 to 7 bases.

16. The nucleic acid construct according to claim 9, wherein a length of the non-complementary region is of 3 bases, and lengths of the complementary regions are each independently of 8 to 11 bases.

17. A nucleic acid construct comprising a 5' untranslated region (UTR) and a 3' UTR that are at least partially complementary to each other, wherein at least one of UTRs includes a non-complementary region and a complementary region with respect to the other UTR, and the non-complementary region and the complementary region are alternately present.

18. The nucleic acid construct according to claim 17, wherein when the non-complementary region is 2 bases, the complementary region is 5 to 7 bases in length in a case of base substitution and 5 to 11 bases in length in a case of base removal.

19. The nucleic acid construct according to claim 17, wherein when the non-complementary region is 3 bases, the complementary region is 8 to 9 bases in length in a case of base substitution and 8 to 11 bases in length in a case of base removal.

20. The nucleic acid construct according to any one of claims 1 to 19, wherein either the 5' UTR or the 3' UTR does not comprise a non-complementary region.

21. The nucleic acid construct according to any one of claims 1 to 20, wherein a change in free energy ($\Delta$G) is a predetermined value or a range thereof.

22. The nucleic acid construct according to claim 21, wherein the predetermined value or the range thereof is a value or range of a change in free energy in a range of a rate of increase of 0% to 60% with respect to an average value of a maximum value and a minimum value of the change in free energy ($\Delta$G).

23. The nucleic acid construct according to claim 22, wherein the predetermined value or the range thereof is a value or range of a change in free energy in a range of a rate of increase of 10% to 50% with respect to an average value of a maximum value and a minimum value of the change in free energy ($\Delta$G).

24. A method of producing a nucleic acid construct, the nucleic acid construct including a 5' untranslated region (UTR) and a 3' UTR that are at least partially complementary to each other, in which at least one of UTRs includes a non-complementary region and a complementary region to the other UTR, the method comprising: a step of designing a plurality of candidate nucleic acid constructs; a step of calculating a change in free energy ($\Delta$G) for the plurality of designed candidate nucleic acid constructs; a step of selecting a candidate nucleic acid construct of which a change in free energy ($\Delta$G) is a predetermined value or a range thereof; and a step of optionally measuring an expression level of the selected candidate nucleic acid construct in cells.

25. A method of producing a nucleic acid construct, the nucleic acid construct including a 5' untranslated region (UTR) and a 3' UTR that are at least partially complementary to each other, in which at least one of UTRs includes a non-complementary region and a complementary region to the other UTR, the method comprising: a step of designing a plurality of candidate nucleic acid constructs; a step of calculating a change in free energy ($\Delta$G) for the plurality of designed candidate nucleic acid constructs; a step of selecting a candidate nucleic acid construct having a value of a change in free energy in a range of a rate of increase of 0% to 60% with respect to an average value of a maximum value and a minimum value of the change in free energy ($\Delta$G); and a step of optionally measuring an expression level of the selected candidate nucleic acid construct in cells.

26. The method according to claim 25, wherein the rate of increase with respect to the average value is 10% to 50%.

27. The nucleic acid construct according to any one of claims 1 to 23, wherein the nucleic acid construct is produced by the method according to any one of claims 24 to 26.

[Fig. 1]

**Fig. 1**

[Fig. 2]

**Fig. 2**

cap   5' UTR

3' UTR

AAAAAAA

ORF

DEGREE OF COMPLEMENTARITY : 100%

GCUCUCUGCUCCUCCUGUUCGACAGUCAGCCGCAUCUUCUUUUGCGUCGCCAGCCGAGCCACAU
CGAGAGACGAGGAGGACAAGCUGUCAGUCGGCGUAGAAGAAAACGCAGCGGUCGGCUCGGUGUA

DEGREE OF COMPLEMENTARITY : 94%

GCUCUCUGCUCCUCCUGUUCGACAGUCAGCCGCAUCUUCUUUUGCGUCGCCAGCCGAGCCACAU
GGAGAGACGAGGAGGAGAAGCUGUCAGUCGGCCUAGAAGAAAACGCAGGGUCGGCUCGGUGUA

DEGREE OF COMPLEMENTARITY : 75%  #1

GCUCUCUGCUCCUCCUGUUCGACAGUCAGCCGCAUCUUCUUUUGCGUCGCCAGCCGAGCCACAU
GGAGUGACCAGGUGGAGAAGUGUGUAGUCGGCUAGUAGAUAACCCAGCGGUCGGCACGGAGUA

DEGREE OF COMPLEMENTARITY : 75%  #2

GCUCUCUGCUCCUCCUGUUCGACAGUCAGCCGCAUCUUCUUUUGCGUCGCCAGCCGAGCCACAU
GUAGAGACCCGGAGGAGUAGCUGUGUGUCGGCCAAGAAGAUUACGCAGCGCGUCGGCACGGUGUA

DEGREE OF COMPLEMENTARITY : 75%  #3

GCUCUCUGCUCCUCCUGUUCGACAGUCAGCCGCAUCUUCUUUUGCGUCGCCAGCCGAGCCACAU
GCUCAGACGAGGAGGAGUUCCUGUCAGUCGGCCAUCAAGAAAACGCAGGCCACGGCUCGGUGUA

DEGREE OF COMPLEMENTARITY : 75%  #4

GCUCUCUGCUCCUCCUGUUCGACAGUCAGCCGCAUCUUCUUUUGCGUCGCCAGCCGAGCCACAU
GCUCUCUGGAGGAGGACAAGCUGUCAGUCGGCGUAGAAGAAAACGCAGCGGUCGGCAGCCACAU

293 CELLS

E2-Crimson/Calcein-AM (AU)

3' UTR  100%  94%  75%_1  75%_2  75%_3  75%_4  Pfizer

[Fig. 3]

## Fig. 3  5' UTR SEQUENCE: GAPDH GENE

### NON-COMPLEMENTARY REGION (WHITE) = 1 BASE

DEGREE OF COMPLEMENTARITY : 100%

GCUCUCUGCUCCUCCUGUUCGACAGUCAGCCGCAUCUUCUUUUGCGUCGCCAGCCGAGCCACAU
CGAGAGACGAGGAGGACAAGCUGUCAGUCGGCGUAGAAGAAAACGCAGCGGUCGGCUCGGUGUA

DEGREE OF COMPLEMENTARITY : 92%   COMPLEMENTARITY/NON-COMPLEMENTARITY : 10 / 1

DEGREE OF COMPLEMENTARITY : 91%   COMPLEMENTARITY/NON-COMPLEMENTARITY : 9 / 1

DEGREE OF COMPLEMENTARITY : 89%   COMPLEMENTARITY/NON-COMPLEMENTARITY : 8 / 1

DEGREE OF COMPLEMENTARITY : 89%   COMPLEMENTARITY/NON-COMPLEMENTARITY : 7 / 1

DEGREE OF COMPLEMENTARITY : 85%   COMPLEMENTARITY/NON-COMPLEMENTARITY : 6 / 1

DEGREE OF COMPLEMENTARITY : 84%   COMPLEMENTARITY/NON-COMPLEMENTARITY : 5 / 1

DEGREE OF COMPLEMENTARITY : 81%   COMPLEMENTARITY/NON-COMPLEMENTARITY : 4 / 1

DEGREE OF COMPLEMENTARITY : 75%   COMPLEMENTARITY/NON-COMPLEMENTARITY : 3 / 1

### NON-COMPLEMENTARY REGION (WHITE) = 2 BASES

DEGREE OF COMPLEMENTARITY : 88%   COMPLEMENTARITY/NON-COMPLEMENTARITY : 13 / 2

DEGREE OF COMPLEMENTARITY : 84%   COMPLEMENTARITY/NON-COMPLEMENTARITY : 10 / 2

DEGREE OF COMPLEMENTARITY : 84%   COMPLEMENTARITY/NON-COMPLEMENTARITY : 9 / 2

DEGREE OF COMPLEMENTARITY : 81%   COMPLEMENTARITY/NON-COMPLEMENTARITY : 8 / 2

DEGREE OF COMPLEMENTARITY : 75%   COMPLEMENTARITY/NON-COMPLEMENTARITY : 6 / 2

### NON-COMPLEMENTARY REGION (WHITE) = 3 BASES

DEGREE OF COMPLEMENTARITY : 91%   COMPLEMENTARITY/NON-COMPLEMENTARITY : 20 / 3

GCUCUCUGCUCCUCCUGUUUGACAGUCAGCCGCAUCUUCUUUUGCGUCGCCAGCCGAGCCACAU
CGAGAGACGAGGAGGACAAGGACUCAGUCGGCGUAGAAGAAAAGCGAGCGGUCGGCUCGGUGUA

DEGREE OF COMPLEMENTARITY : 86%   COMPLEMENTARITY/NON-COMPLEMENTARITY : 14 / 3

DEGREE OF COMPLEMENTARITY : 81%   COMPLEMENTARITY/NON-COMPLEMENTARITY : 11 / 3

[Fig. 4]

**Fig. 4**

[Fig. 5]

## Fig. 5  5' UTR SEQUENCE: Pfizer SEQUENCE

[Fig. 6]

Fig. 6

[Fig. 7]

**Fig. 7**    5' UTR SEQUENCE: HSD17B4 GENE

[Fig. 8]

**Fig. 8**

[Fig. 9]

## Fig. 9  5' UTR SEQUENCE: GAPDH GENE

DEGREE OF COMPLEMENTARITY : 92%    COMPLEMENTARITY/ NON-COMPLEMENTARITY : 10 / 1

DEGREE OF COMPLEMENTARITY : 91%    COMPLEMENTARITY/ NON-COMPLEMENTARITY : 9 / 1

DEGREE OF COMPLEMENTARITY : 89%    COMPLEMENTARITY/ NON-COMPLEMENTARITY : 8 / 1

DEGREE OF COMPLEMENTARITY : 89%    COMPLEMENTARITY/ NON-COMPLEMENTARITY : 7 / 1

DEGREE OF COMPLEMENTARITY : 85%    COMPLEMENTARITY/ NON-COMPLEMENTARITY : 6 / 1

DEGREE OF COMPLEMENTARITY : 84%    COMPLEMENTARITY/ NON-COMPLEMENTARITY : 5 / 1

DEGREE OF COMPLEMENTARITY : 81%    COMPLEMENTARITY/ NON-COMPLEMENTARITY : 4 / 1

DEGREE OF COMPLEMENTARITY : 80%    COMPLEMENTARITY/ NON-COMPLEMENTARITY : 4, 3 / 1

DEGREE OF COMPLEMENTARITY : 78%    COMPLEMENTARITY/ NON-COMPLEMENTARITY : 4, 3 / 1

DEGREE OF COMPLEMENTARITY : 78%    COMPLEMENTARITY/ NON-COMPLEMENTARITY : 4, 3 / 1

DEGREE OF COMPLEMENTARITY : 78%    COMPLEMENTARITY/ NON-COMPLEMENTARITY : 4, 3 / 1

DEGREE OF COMPLEMENTARITY : 77%    COMPLEMENTARITY/ NON-COMPLEMENTARITY : 4, 3 / 1

DEGREE OF COMPLEMENTARITY : 77%    COMPLEMENTARITY/ NON-COMPLEMENTARITY : 4, 3 / 1

DEGREE OF COMPLEMENTARITY : 77%    COMPLEMENTARITY/ NON-COMPLEMENTARITY : 4, 3 / 1

DEGREE OF COMPLEMENTARITY : 75%    COMPLEMENTARITY/ NON-COMPLEMENTARITY : 3 / 1

[Fig. 10]

Fig. 10

[Fig. 11]

**Fig. 11** FITTING TO SIGMOID CURVE

PARAMETER c:
VALUE OF FREE ENERGY
(x-AXIS) AT INFLECTION
POINT OF SIGMOID
CURVE

| 5'UTR | | GAPDH | HSD17B4 | Pfizer |
|---|---|---|---|---|
| PARAMETERS | a | 2.03827 | 1.58691 | 1.52629 |
| | b | 10.45743 | 108.51179 | 15.34508 |
| | c | -88.0351 | -83.27308 | -51.79991 |
| | d | 0.29640 | 0.41246 | 0.38911 |

| 5' UTR | R2 CORRELATION COEFFICIENT |
|---|---|
| GAPDH | 0.5935 |
| HSD17B4 | 0.7697 |
| Pfizer | 0.8009 |

[Fig. 12]

**Fig. 12**

| 5'UTR | | GAPDH | HSD17B4 | Pfizer |
|---|---|---|---|---|
| MINIMUM VALUE OF CHANGE IN FREE ENERGY * | | -141.6 | -134.4 | -92.3 |
| MAXIMUM VALUE OF CHANGE IN FREE ENERGY * * | | -24.6 | -36.9 | -19.7 |
| AVERAGE OF MINIMUM AND MAXIMUM VALUES OF CHANGE IN FREE ENERGY | | -83.1 | -85.65 | -56.0 |
| SIGMOID INFLECTION POINT | | -88.0 | -83.4 | -51.8 |
| INFLECTION POINT-MINIMUM VALUE OF CHANGE IN FREE ENERGY RANGE | 10% | -81.7 | -78.6 | -48.6 |
| | 50% | -56.3 | -60.1 | -35.8 |
| EXPRESSION LEVEL | MAXIMUM VALUE | -71.7 | -74.4 | -37.9 |
| | 2nd | -65 | -82.2 | -33.1 |
| | 3rd | -62.9 | -61.4 | -44 |

[Fig. 13]

**Fig. 13**

| 5'UTR | | GAPDH | HSD17B4 | Pfizer |
|---|---|---|---|---|
| MINIMUM VALUE OF CHANGE IN FREE ENERGY ∗ | | -141.6 | -134.4 | -92.3 |
| MAXIMUM VALUE OF CHANGE IN FREE ENERGY ∗∗ | | -24.6 | -36.9 | -19.7 |
| AVERAGE OF MINIMUM AND MAXIMUM VALUES OF CHANGE IN FREE ENERGY | | -83.1 | -85.7 | -56.0 |
| SIGMOID INFLECTION POINT | | -88.0 | -83.4 | -51.8 |
| ERROR | | 4.9 | 2.3 | 4.2 |
| EXPRESSION LEVEL | 1st | -71.7 | -74.4 | -37.9 |
| | 2nd | -65 | -82.2 | -33.1 |
| | 3rd | -62.9 | -61.4 | -44 |

∗ MINIMUM VALUE OF CHANGE IN FREE ENERGY: DEGREE OF COMPLEMENTARITY OF 100%

∗∗ MAXIMUM VALUE OF CHANGE IN FREE ENERGY: SEQUENCE IN WHICH ALL BASES IN 3' UTR ARE SUBJECTED TO MISMATCHED SUBSTITUTION

[Fig. 14]

**Fig.14**

[Fig. 15]

**Fig.15**

[Fig. 16]

Fig.16

[Fig. 17A]

(1) BASE SUBSTITUTION

(2) 3' UTR BASE REMOVAL

(3) 5' UTR BASE REMOVAL

(4) BASE SUBSTITUTION AND REMOVAL MIXTURE

(4-1) BASE SUBSTITUTION AND REMOVAL MIXTURE ON 3' UTR

(4-2) BASE SUBSTITUTION AND REMOVAL MIXTURE ON 5' UTR

(4-3) BASE SUBSTITUTION AND REMOVAL MIXTURE ON 5'/3' UTR

[Fig. 17B]

(5) 3' UTR BASE ADDITION

(6) 5' UTR BASE ADDITION

[Fig. 18A]

5' UTR SEQUENCE: Pfizer SEQUENCE  (-: BASE REMOVAL SITE)

## （2）3' UTR BASE REMOVAL

NON-COMPLEMENTARY REGION (WHITE)
= 1 BASE

NON-COMPLEMENTARY REGION (WHITE)
= 2 BASES

NON-COMPLEMENTARY REGION (WHITE)
= 3 BASES

[Fig. 18B]

# 5' UTR SEQUENCE: Pfizer SEQUENCE   (-: BASE REMOVAL SITE)

## (3) 5' UTR BASE REMOVAL

## (4) BASE SUBSTITUTION AND REMOVAL MIXTURE

(4－1) BASE SUBSTITUTION AND REMOVAL MIXTURE ON 3' UTR

(4－2) BASE SUBSTITUTION AND REMOVAL MIXTURE ON 5' UTR

(4－3) BASE SUBSTITUTION AND REMOVAL MIXTURE ON 5'/3' UTR

[Fig. 18C]

## 5' UTR SEQUENCE: Pfizer SEQUENCE    (-: BASE REMOVAL SITE)

### (5) 3' UTR BASE ADDITION

DEGREE OF COMPLEMENTARITY :91%    COMPLEMENTARITY/ NON-COMPLEMENTARITY :8/1

```
GAGAAUAAACUAGUAUUCUUCUGGUCCCCACAGACUCAGAGAGAACCC
CUCUUAUUUGAUCAUAAGAAGACCAGGGGUGUCUGAGUCUCUCUUGGG
       A        U        G        C        A
```

DEGREE OF COMPLEMENTARITY :83%    COMPLEMENTARITY/ NON-COMPLEMENTARITY :8/2

```
GAGAAUAAACUAGUAUUCUUCUGGUCCCCACAGACUCAGAGAGAACCC
CUCUUAUUUGAUCAUAAGAAGACCAGGGGUGUCUGAGUCUCUCUUGGG
      AU      GC      AG      UA      UG
```

DEGREE OF COMPLEMENTARITY :76%    COMPLEMENTARITY/ NON-COMPLEMENTARITY :8/3

```
GAGAAUAAACUAGUAUUCUUCUGGUCCCCACAGACUCAGAGAGAACCC
CUCUUAUUUGAUCAUAAGAAGACCAGGGGUGUCUGAGUCUCUCUUGGG
     GAU     AUC     UGA     CUG     AGC
```

### (6) 5' UTR BASE ADDITION

DEGREE OF COMPLEMENTARITY :91%    COMPLEMENTARITY/ NON-COMPLEMENTARITY :8/1

```
     A        U        G        C        A
GAGAAUAAACUAGUAUUCUUCUGGUCCCCACACACUCAGAGAGAACCC
CUCUUAUUUGAUCAUAAGAAGACCAGGGGUGUCUGAGUCUCUCUUGGG
```

DEGREE OF COMPLEMENTARITY :83%    COMPLEMENTARITY/ NON-COMPLEMENTARITY :8/2

```
    AU      GC      AG      UA      UG
GAGAAUAAACUAGUAUUCUUCUGGUCCCCACAGACUCAGAGAGAACCC
CUCUUAUUUGAUCAUAAGAAGACCAGGGGUGUCUGAGUCUCUCUUGGG
```

DEGREE OF COMPLEMENTARITY :76%    COMPLEMENTARITY/ NON-COMPLEMENTARITY :8/3

```
   GAU     AUC     UGA     CUG     AGC
GAGAAUAAACUAGUAUUCUUCUGGUCCCCACAGACUCAGAGAGAACCC
CUCUUAUUUGAUCAUAAGAAGACCAGGGGUGUCUGAGUCUCUCUUGGG
```

[Fig. 19]

Fig.19

[Fig. 20]

Fig.20

[Fig. 21]

**Fig.21**

[Fig. 22]

**Fig.22**

| COMPLEMENTARY (BASE) | 14 13 12 11 10 9 8 | Pfizer |
| NON-COMPLEMENTARY (BASE) | 3 | |
| DEGREE OF COMPLEMENTARITY (%) | 88 81 81 81 81 75 75 | |

[Fig. 23]

SARS CoV2 spike-
E2Crimson FUSION
PROTEIN(4.7kb)

**Fig.23**

[Fig. 24]

## 5' UTR SEQUENCE: Pfizer SEQUENCE  (-: BASE REMOVAL SITE)

CASE IN WHICH NON-COMPLEMENTARY REGIONS HAVING DIFFERENT NUMBERS OF BASES ARE MIXED

(1) CASE IN WHICH NUMBER OF BASES OF NON-COMPLEMENTARY REGION IS SAME BETWEEN 5' UTR AND 3' UTR

(2) CASE IN WHICH NUMBER OF BASES OF NON-COMPLEMENTARY REGION DIFFERS BETWEEN 5' UTR AND 3' UTR

## INTERNATIONAL SEARCH REPORT

| International application No. |
| --- |
| **PCT/JP2024/002945** |

| A. | CLASSIFICATION OF SUBJECT MATTER |
| --- | --- |

***C12N 15/11***(2006.01)i
FI:   C12N15/11 Z ZNA

According to International Patent Classification (IPC) or to both national classification and IPC

| B. | FIELDS SEARCHED |
| --- | --- |

Minimum documentation searched (classification system followed by classification symbols)

C12N15/11

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | WO 2021/075567 A1 (NAGOYA CITY UNIVERSITY) 22 April 2021 (2021-04-22)<br>claims, examples | 1-20, 27 |
| Y | claims, examples | 21-27 |
| Y | 田中宏昌他. R-STEINER: mRNA高翻訳化のための5'UTR生成手法. DEIM Forum 2018. 2018, F5-4, non-official translation (TANAKA, Hiromasa et al. R-STEINER: 5'UTR Generation Method for High mRNA Translation.)<br>"4.R-STEINER" column | 21-27 |
| A | "4.R-STEINER" column | 1-20 |
| A | 稲垣雅仁他. 翻訳反応を最大化するための合成mRNAの分子設計. Drug Delivery System. 2022, vol. 37, no. 3, pp.196-208, (INAGAKI, Masahito et al. Design of Synthetic mRNAs for Highly Efficient Translation.) | 1-27 |

☐ Further documents are listed in the continuation of Box C.      ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **19 March 2024** | **02 April 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

## INTERNATIONAL SEARCH REPORT

International application No.

**PCT/JP2024/002945**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT
### Information on patent family members

| International application No. |
| --- |
| **PCT/JP2024/002945** |

| Patent document cited in search report | Publication date (day/month/year) | Patent family member(s) | Publication date (day/month/year) |
| --- | --- | --- | --- |
| WO 2021/075567 A1 | 22 April 2021 | EP 4047084 A1 claims, examples | |

Form PCT/ISA/210 (patent family annex) (July 2022)

**REFERENCES CITED IN THE DESCRIPTION**

**Patent documents cited in the description**

- US 5908845 A
- JP 2021501572 A
- JP 2015517803 A
- JP 2022164843 A
- JP 2023013290 A

**Non-patent literature cited in the description**

- **MICHAEL ZUKER**. Mfold web server for nucleic acid folding and hybridization prediction. *Nucleic Acids Res.*, 01 July 2003, vol. 31 (13), 3406-15
- **P CLOTE**. RNALOSS: a web server for RNA locally optimal secondary structures. *Nucleic Acids Res.*, 01 July 2005, vol. 33, W600-4
- **SAMBROOK, J. et al.** Molecular Cloning: A Laboratory Manual.. Cold Spring Harbor Laboratory Press, 1989